# EUROPEAN PATENT APPLICATION

(11) **EP 0 804 883 A2**
(43) Date of publication of application: **05.11.1997**
(21) Application number: 97303014.1
(22) Date of filing: 01.05.1997
(51) Int. Cl.: A23K 3/02, A23K 1/18, A01H 5/10, A61K 38/27

(54) **Animal feed**

(30) Priority: 01.05.1996 US 641486; 19.08.1996 US 699560; 06.02.1997 US 795549
(71) Applicant: Cargill Incorporated, Wayzata, Minnesota 55391 (US)
(72) Inventor: Beck, James F., Marshall, Michigan 49068 (US); Storck, Dale H., Sugar Grove, Illinois 60554 (US); Casper, David P., Elk River, Minnesota 55330 (US)
(74) Representative: Dzieglewska, Hanna Eva

(57) **Abstract**

A ruminant animal feed comprising a forage component with about 20% to about 100% corn silage produced from corn plants exhibiting a *bm* phenotype and a feed composition component and a method of producing the same is disclosed. A method of enhancing milk production in ruminant animals, especially dairy cattle, by feeding them with the animal feed of the invention, optionally co-administering somatotropin, is also disclosed.

## Description

This invention relates to increasing milk production in ruminant animals such as dairy cows. More particularly the invention relates to increasing milk production in animals such as dairy cows by feeding a ration comprising silage from corn plants exhibiting a brown midrib phenotype.

Corn plants (Zea mays L.) are bred by both self-pollination and cross-pollination techniques. Corn is a monoecious plant, i.e., each plant has separate male and female flowers on the same plant, located on the tassel and ear, respectively. Natural pollination occurs in corn when pollen is shed from tassels and contacts silks of the same plant or a different plant that protrude from tops of the developing ears. Methods and techniques for the development of inbred corn lines and hybrid corn varieties are known in the art. Hallauer, A., Maize, in Principles of Cultivar Development, Vol. 2, Fehr. W. ed. pp. 249-294, Macmillan, New York, (1987). Currently, many hybrid corn varieties are produced by crossing two inbred lines to product F₁ hybrid progeny. The F₁ plants exhibit heterosis, or hybrid vigor, resulting in plants having high yield and superior agronomic performance in the hybrid combination. The production and development of inbred corn lines and hybrid corn varieties are discussed in, for example, U.S. Patent Nos. 5,367,109 and 5,495,067, which are incorporated herein by reference.

Research studies on maize have resulted in the identification of numerous genetic loci. See, e.g., the maize genetic database on the Worldwide Web at http://teosinte.agron. missour.edu/top.html.

To supply needed nutrients for increased milk production by lactating dairy cattle is an ongoing challenge facing the dairy industry. This challenge is complicated by the fact that, even though a dairy cow's diet may meet the National Research Council recommended nutrient requirements, the diet may still lack some nutrients at increased levels required for higher milk production. One reason for this difficulty in meeting nutrient requirements is the complexity of the digestive system of ruminants such as dairy cattle.

In cattle, ingested feed first passes into the reticulorumen, where it is subject to anaerobic microbial fermentation. Microbial fermentation begins the digestive process and gives a ruminant the ability to utilize fibrous feeds, in contrast to monogastric animals. Ruminants meet their nutrient needs by utilizing the by-products of microbial fermentation, along with any undigested feed residues and the resultant microbial mass that passes from the rumen.

Anaerobic microbial fermentation is an advantage to ruminants because it allows them to benefit from feeds which cannot be utilized by non-ruminants. However, microbial activity limits the ability to provide supplemental nutrients to a ruminant animal, because many desirable nutrients, such as proteins, amino acids and digestible fiber, will be metabolized by microbes before the nutrients reach a site where they can be absorbed and utilized by the ruminant.

Attempts have been made to increase milk production in dairy cattle by manipulating the feed ration. For example, rations containing silage derived from corn plants carrying a brown midrib *(bm)* mutation have been fed to cattle. Stallings, C. et al., J. Dairy Sci., 65:1945-1949 (1982); Block, E. et al., J. Dairy Sci. 64:1813-1825 (1981); Keith, E. et al., J. Dairy Sci. 62:788 (1979). The *bm* gene decreases and alters the lignin content in the vegetative parts of such corn plants; silage made from such plants has increased fiber digestibility compared to silage from corn plants not exhibiting the brown midrib phenotype. In general, these studies indicated that there was no increase in milk production in cows fed silage from *bm* corn. It was concluded that the cows fed a diet containing *bm* silage generally partitioned the nutrients into meat or fat body tissues rather than milk production. Barriere et al., Agronomie 13:865-876 (1993).

Attempts have been made to increase the efficiency of feed utilization and milk production by using various formulations and feed supplements. Despite continued improvement in the development of dairy cattle feed rations, it is desirable to further increase the efficiency of feed utilization and milk production by dairy cattle.

It has now surprisingly been found that silage from *bm* corn may be used to increase the milk production of ruminant animals when combined to make an animal feed with defined constituents.

Thus, viewed from one aspect the present invention provides a ruminant animal feed comprising a combination of:
a forage component comprising from about 20% to about 60% of said feed on a dry matter basis, said forage component comprising from about 20% to about 100% corn silage on a dry matter basis produced from corn plants exhibiting a brown midrib *(bm)* phenotype, said silage having an *in vitro* neutral detergent fibre digestibility of about 44% to about 70%; and
a feed composition component;
said animal feed having a fibre content of about 20% to about 40%. Preferably the animal feed of the invention is in the form of a total feed ration, preferably a total dairy cattle feed ration.

The ration can have a crude protein content of from about 17% to about 21% on a dry matter basis. About 30% to about 50% of the crude protein is soluble protein.

The neutral detergent fiber digestibility can be from about 6% to about 20% greater than the neutral detergent fiber digestibility of corn silage produced from corresponding isogenic normal corn plants. The silage can have a whole plant *in vitro* digestibility from about 65% to about 85%.

The corn plants can comprise F₁ hybrid plants. The brown midrib phenotype can be the result of homozygosity at the *bm3* gene locus.

Viewed from a further aspect the invention provides a method of enhancing milk production in a ruminant animal, such as cattle, sheep or goats, preferably in a dairy cow, comprising the step of feeding said animal an animal feed as defined hereinbefore. Alternatively viewed the present invention provides an animal feed as described herein for enhancing milk production in a ruminant animal. Alternatively, the use of a forage component or a feed composition component as described herein for the preparation of an animal feed as described herein for enhancing milk production in a ruminant animal, is also provided.

The method can further comprise the step of administering a biologically active somatotropin to the animal, e.g. a cow under conditions delivering an effective amount of the somatotropin to the animal during a selected period. The somatotropin can be administered as a prolonged release dose, for example, a dose that is effective for at least 7 days. The conditions can comprise delivering the somatotropin to the circulatory system of the animal.

In some embodiments, the forage component comprises from about 40% to about 60% of the ration, and the *bm* corn silage comprises from about 40% to about 80% of the forage component. In some embodiments, the forage component comprises from about 45% to about 55% of the ration and the *bm* corn silage comprises from about 50% to about 80% of the forage component.

The present invention further extends to a method of producing a total ruminant animal feed as defined herein comprising the steps of a) obtaining a corn silage produced from corn plants exhibiting a *bm* phenotype; and b) formulating a total ruminant animal feed comprising a combination of a forage component and a feed composition component as defined herein.

Preferably in this method said corn plants are grown from a substantially homogenous assemblage of corn seeds which are homozygous for at least one *bm* allele.

According to a yet further aspect of the invention we provide a pack for a ruminant animal feed comprising a forage component and separately a feed composition component as defined herein as a combined preparation for simultaneous, separate or sequential use to enhance milk production in said animal. Such a pack may additionally comprise a biologically active somatotropin. Novel plants and seeds described herein also form aspects of the invention. The seeds of the invention may be viable or alternatively non-viable, such as crushed, milled or pressed seeds. Silage produced from the novel seeds and plants of the invention form yet further aspects of the invention.

An article of manufacture is disclosed, comprising packaging material, a substantially homogenous assemblage of F₁ hybrid corn seeds within the packaging material and a package label accompanying the packaging material. The seeds can be homozygous for *bm3.* The package label indicates that the seeds are effective for producing silage that increases milk production when fed to lactating dairy cattle.

The package label can indicate that the seeds are effective for producing silage having an *in vitro* neutral detergent fiber digestibility of from about 44% to about 70% and a whole plant *in vitro* digestibility of about 65% to about 85% determined after ensiling the plants.

The package label can also indicate that the seeds are effective for producing silage that increases milk production when fed to lactating dairy cows in a ration having a fiber content of about 20% to about 40%. Such a ration comprises about 20% to about 60% of a forage component, with the remainder being a feed composition. The forage component comprises from about 20% to about 100% corn silage produced from corn plants homozygous for *bm3*. The package label can also indicate that the ration has a crude protein content of from about 17% to about 21% on a dry matter basis and about 35% to about 50% of the crude protein is soluble protein.

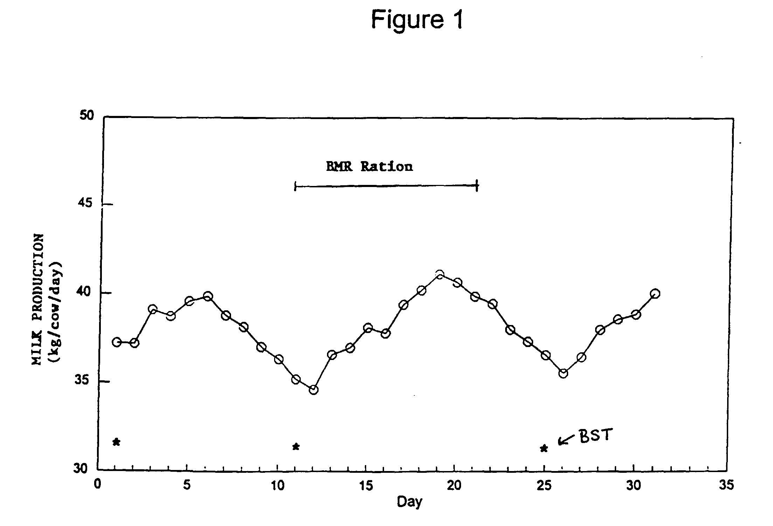

Figure 1 shows herd milk production on a daily basis in a switchback trial in which normal or BMR silage was fed in conjunction with bovine somatotropin administration.

A feed ration has been discovered that comprises a corn silage produced from corn plants exhibiting a brown midrib phenotype. Feeding of such silage to dairy cattle results in unexpected improvements in milk production.

A silage component of the feed ration is produced from corn plants displaying a brown midrib *(bm)* phenotype. The *bm* phenotype is exhibited by plants homozygous for a mutant allele at the *bm1, bm2, bm3* or *bm4* loci. In some embodiments, such plants may display the brown midrib phenotype due to homozygosity at more than one of the *bm* loci. Mutant *bm* alleles are known to reduce and alter the lignin content in plants homozygous for such alleles. The lignin content may be reduced 20%, 30%, or up to about 45% compared to corn of the same genetic background but having a wild-type *Bm* gene.

Corn inbreds and hybrids carrying *bm* alleles and displaying the *bm* phenotype can be produced by corn breeding methods such as conversion programs or recurrent selection. In one embodiment, a corn inbred line is converted to the *bm* phenotype in a breeding program initiated from the F₁ progeny of a cross between a plant of a first inbred (wild-type for the *bm* phenotype) and plants of a second line carrying the desired *bm* allele. F₁ plants are backcrossed to the first inbred line until an inbred line is obtained that has substantially the same genotype as the original inbred line except for the replacement of the wild-type *Bm* gene by the mutant *bm* gene. Conversion programs, recurrent selection programs, pedigree breeding programs, breeding programs using synthetics and other breeding methods are described in, e.g., Hallauer, et al. in Corn and Corn Improvement, Sprague et al., eds. pp. 463-564 (1988).

In addition to selecting and identifying plants containing a mutant *bm* gene, it is desirable to select concomitantly for plants having superior agronomic and yield performance characteristics.

Techniques for identifying plants displaying the brown midrib phenotype are known in the art. For example, the underside of leaves may be examined at 10-14 days before tassel emergence (4-6 leaf stage, 0.6-1 meter height) for the appearance of a golden-brown or reddish-brown color on the midrib. Plants may also be examined at maturity by removing a leaf sheath and examining the stalk. The stalk has a golden-brown or reddish-brown color if the brown midrib phenotype is expressed. Brown pigment is also present in the cob and in the roots. Because the *bm* phenotype is recessive, the presence of the *bm* gene in heterozygotes can be determined by performing a self and evaluating the selfed progeny for the expected 3:1 segregation ratio. Alternatively, marker-assisted breeding techniques may be used, e.g., restriction fragment length polymorphisms (RFLP), simple sequence repeats (SSR), microsatellite markers or PCR markers. Marker-assisted breeding techniques are useful, in that plants heterozygous for the *bm* allele can be identified without the necessity for evaluating phenotypic ratios in selfed progeny.

Once inbreds having the *bm* phenotype and desired performance characteristics have been identified, each inbred is evaluated for the development of appropriate hybrid combinations by test crosses or top crosses to another inbred displaying the *bm* phenotype.

Suitable hybrids are selected to have certain desirable agronomic characteristics. Such characteristics include, for example, satisfactory disease resistance or tolerance, satisfactory insect resistance or tolerance and satisfactory seedling vigor. Such general characteristics are desired in all types of corn hybrids regardless of the intended use of the hybrid; the best available performance with respect to these characteristics will be incorporated into a *bm* hybrid. Methods and tests for identifying inbreds having the desired general agronomic performance characteristics in hybrid combination are known in the art.

In addition, hybrids are developed that are adapted for use in short, medium or long growing seasons according to a relative maturity rating system such as the Minnesota Maturity Rating (MMR). See, e.g., U.S. Patent 5,495,067, incorporated herein by reference.

However, certain other characteristics are of particular relevance in selecting suitable *bm* inbreds and hybrids. One relevant characteristic is the forage yield of the hybrid. The forage yield of a suitable *bm* hybrid is from about 20 to about 28 Tons per acre (adjusted to 70% moisture), preferably from about 22 Tons per acre to about 28 Tons per acre, more preferably from about 24 Tons per acre to about 28 Tons per acre. The forage yield can be from about 25 Tons/acre to about 32 Tons/acre in newer hybrids that are converted to express the *bm* phenotype.

A *bm* hybrid typically has a decrease in forage yield compared to the forage yield of its isogenic counterpart lacking the *bm* phenotype. However, preferred hybrids have a forage yield decrease of less than 15% compared to their isogenic counterparts, preferably a decrease of about 10% or less.

Another relevant characteristic is the *in vitro* digestibility of corn silage made from a *bm* hybrid as determined after about 30 days of fermentation. *In vitro* true digestibility can be measured by determining neutral detergent fiber (NDF) digestibility. See, e.g., Goering, H. and Van Soest, P., Forage Fiber analyses in Agriculture Handbook 379, U.S. Department of Agriculture, Washington, D.C., pp. 1-20 (1975). *In vitro* NDF digestibility is also referred to herein as *in vitro* cell wall digestibility (IVCWD). Neutral detergent fiber is a measure of the cellulose, hemicellulose and lignin fractions of silage and constitutes from about 35 percent to about 55 percent of the silage dry matter, generally from about 40 percent to about 50 percent. Preferred *bm* hybrids have an *in vitro* NDF digestibility of from about 44 percent to about 70 percent, e.g., from about 44 percent to about 60 percent, or from about 47 percent to about 55 percent. In some embodiments, the *in vitro* NDF digestibility is from about 44% to about 50%. The NDF digestibility of *bm* hybrids preferably is about 6 to about 20 percentage units greater than the corresponding isogenic normal hybrid (which is homozygous for *Bm3),* e.g., about 6 to about 15 percentage units greater.

*In vitro* digestibility can also be measured on the whole plant after about 30 days of fermentation. Whole plant *in vitro* digestibility is also referred to herein as IVTD and is based on the Tilley and Terry fermentation method. Tilley, J. and Terry, R. J. Brit. Grassland Soc. 18:104-111 (1963). Suitable hybrids have an IVTD value of from about 65% to about 85%, e.g., from about 70% to about 85%, or from about 74% to about 85%. In certain embodiments, the IVTD value of *bm* hybrids is from about 74 percent to about 80 percent. The IVTD value of a *bm* hybrid is about 2 to about 7 percentage units greater than the IVTD value of the corresponding isogenic normal hybrid.

*In vitro* NDF digestibility and IVTD can be measured, for example, by collecting fresh-cut plant material and ensiling it in mini-silo fermentation canisters. The material may be reshred prior to ensiling to provide more uniform fermentation. After about 30 days, the pH of the fermented material is stable and *in vitro NDF* Digestibility and IVTD are determined as described in Goering, H. and Van Soest, P., *supra,* except that a 30 hour in *vitro* fermentation is performed rather than a 48 hour *in vitro* fermentation.

The forage yields, IVCWD values and IVTD values of the novel *bm* hybrids disclosed herein are useful in determining the suitability of such hybrids for inclusion in a dairy cattle feed ration.

Once inbreds having the *bm* phenotype and desired performance characteristics have been identified, each inbred is evaluated for the development of appropriate hybrid combinations by test crosses or top crosses to another inbred displaying the *bm* phenotype.

Examples of corn inbreds suitable for producing *bm* corn hybrids include, without limitation, inbreds *AR5252bm3, 7675bm3, 7677bm3, AR5251bm3* and *AR5651bm3.* The inbred lines *AR5252bm3, AR5251bm3, AR5651bm3, AR5551bm3, AR5153bm3, AR5253bm3* and *AR5654bm3* are available from the applicant on request. This however in no way provides a licence of any sort to perform the invention as claimed.

The invention may readily be performed using materials available and known in the art. For example, other corn seeds possessing mutant *bm* alleles are available from various universities and seed stock centers such as Maize Genetics Cooperation - Stock Center, University of Illinois, Department of Crop Science, Urbana, Illinois, USA. These seeds can also be used to initiate a *bm* breeding program.

The invention may alternatively be performed by transfection of appropriate plants to produce antisense molecules to the *Bm* gene whose sequence is known.

An inbred line according to the invention preferably is homozygous for a *bm3* allele. Certain *bm3* alleles have been sequenced, e.g., the *bm3-1* and *bm3-2* alleles. Vignols et al. Plant Cell 7:407-416 (1995). Alleles that have a deletion, e.g., a deletion similar to that in *bm3-2,* are preferred because such alleles are less likely to revert to wild-type.

It is known in the art that maize germplasm can be divided into a number of distinct heterotic groupings. Such groups include Reid Yellow Dent, Lancaster Sure Crop and subgroups such as Iowa Stiff Stalk Synthetic (Reid Yellow Dent) and Oh43 (Lancaster Sure Crop). One important aspect of a maize breeding program is the identification of the heterotic group to which a particular inbred belongs. By identifying the heterotic group or subgroup, it becomes possible to more clearly determine the appropriate types of crosses, in order to obtain sufficient levels of heterosis or hybrid vigor. Because of the large number of possible heterotic groupings to which a given inbred can belong, it is useful to ascertain which heterotic groups can be most advantageously used to form a hybrid combination from brown midrib inbreds. Inbreds having the mutant *bm* phenotype have not been tested in all possible heterotic groups for the effect of the *bm* phenotype on expression of the mutant trait and the effect on other agronomic traits.

Once a suitable pair of inbred lines that provide the desired performance in hybrid combination have been identified, production of F₁ hybrid *bm* seed is undertaken. Typically, a substantially uniform assemblage of F₁ *bm* hybrid corn seeds is conditioned and bagged in packaging material by means known in the art to form an article of manufacture. Alternatively, Such a novel bag of seed has a package label accompanying the bag, e.g., a tag or label secured to the packaging material, a label printed on the packaging material or a label inserted within the bag. The package label indicates that the seeds therein are effective for producing silage that can be fed to dairy cattle. Preferably, the package label indicates that the resulting silage is to be combined with a feed composition component as disclosed herein. The package label may indicate that silage resulting from seeds contained therein is effective for increasing milk production when fed to lactating cows as disclosed herein.

The novel corn seed is planted and cultivated according to standard agronomic practices in the geographic area to which the hybrid is adapted. Growers typically take into account soil fertility, crop rotation practices and other factors specific to the locale in which the hybrid corn is being grown.

Corn plants can be grown to maturity and the seeds produced thereon harvested for use as grain. However, above-ground parts of corn plants disclosed herein preferably are harvested after grain fill, but before drydown. Typically, plants are harvested using a mechanical forage harvester which chops the above-ground portion of the plant into small pieces. Harvesting typically is based on the stage of seed maturity and occurs about the stage at which the color line is halfway down the kernel. The precise harvest time will depend, of course, upon geographical location and seasonal factors such as the weather. The chopped material is then ensiled by techniques known in the art, e.g., in trenches or in cdncrete stave silos. Microbial inoculants and/or preservatives may be added to promote silage formation, if desired.

Corn silage from *bm* plants is fed to ruminant animals, such as dairy cattle as a total animal feed ration, such as a total dairy cattle feed ration (also referred to herein as a total mixed ration or total ration) comprising *bm* silage and a feed composition. The nutrient composition of the silage can be determined, e.g. percent by dry matter, percent NDF, percent CP, *in vitro* NDF, digestibility and IVTD. Based on this information, a total animal (e.g. dairy cattle) feed ration is formulated.

A feed composition component of the invention may be a complete feed form, a concentrate form, blender form or base mix form. By complete feed form it is meant that the feed represents the cow's entire grain ration. By concentrate form it is meant that the feed will be used as the primary supplemental protein source and would normally be fed with grain to meet an animal's protein needs. By blender form it is meant that the feed will be mixed with approximately a 50:50 ratio with grain to form the complete grain ration. The base mix form is similar to the concentrate form, but is typically higher in protein content and is used at lower inclusion rates. The base mix form will be a primary, but often not the sole source of supplemental protein.

For example, a complete feed form composition may contain wheat middlings, corn, meat and bone meal, soybean meal, salt, macro-minerals, trace minerals and vitamins. Alternative or optional ingredients commonly include, but are not restricted to, fat, sunflower meal, feather meal, malt sprouts, distillers' grains, canola meal and soybean hulls. Other alternative or optional protein sources include, for example, blood meal, corn gluten meal, peanut meal, cottonseed meal, soybeans (extruded or roasted), wheat bran and high fat rice bran.

A concentrate form composition, a blender form composition or a base mix form composition can be prepared by those of skill in the art, based upon the complete feed composition discussed above. Grains fed with the blender, concentrate and base mix forms of the composition can include, but are not limited to, corn, barley, oats, millet, rice, sorghum and wheat. Intake of the total grain ration will typically range from 2.7 to about 17 kilograms (kg) per day.

The nutrient composition of total dairy cattle feed ration comprising *bm* silage contains crude protein (CP) at a relatively high level in order to increase milk production. On a dry matter basis, CP is about 17% or greater, preferably from about 18% to about 21%. If there are reproductive difficulties, the amount of CP may be reduced. About 30% to about 50% of the CP is soluble protein (also referred to as SP, degradable intake protein, or DIP), preferably about 35% to about 50%, more preferably about 40% to about 45%. A total ration having a CP level at the lower end of the range of values results in greater weight gain and smaller increases in milk production. On the other hand, a ration with a CP level at the upper end of the range of values results in smaller weight gains and greater increases in milk production. About 20% to about 40% of the CP in the total ration is rumen undegraded protein (RUP, undegradable intake protein, or UIP), preferably from about 25% to about 40%, more preferably from about 25% to about 35%.

The ADF content of a total ration preferably is about 18% or greater, e.g., about 18% to about 24%, or about 19% to about 22%. The fat content of a total ration typically is from about 4% to about 8%, e.g., about 4% to about 6%. The non-fiber carbohydrate (NFC) content of a total ration is typically from about 36% to 46% on a dry matter basis, e.g., about 39% to about 43%.

The forage component in the total ration typically constitutes about 20% to about 60% of the ration on a dry matter basis and is added to achieve a fiber content of about 20% to about 40%, preferably from about 25% to about 35%. These values are appropriate for cows in early lactation; mid-lactation cows may have a higher forage/concentrate ratio resulting in, for example, about 30% to about 40% fiber content. The *bm* silage comprises from about 20% to about 100% of the forage on a dry matter basis, e.g., from about 25% to about 90%, or from about 40% to about 90%, or from about 70% to about 90%. Although *bm* silage may comprise 100% of the forage component in a ration, higher amounts of a protein source such as soybean meal will need to be included; doing so increases the overall cost of the diet. Therefore, it can be more cost-effective to include *bm* silage at less than 100% of the forage component.

Sources that may be used to complete the forage component of the ration include, but are not limited to, corn silage from non-*bm* corn plants, alfalfa haylage, grass silages (e.g., sudangrass, orchardgrass or sorghum-based silage), grass hays (e.g., sudangrass or orchardgrass) and alfalfa or clover hay. Such other forages are known in the art.

The silage component and the feed composition component are combined and fed to ruminant animals, e.g. dairy cattle, under generally accepted management conditions. For example, typical dairy cow management conditions include known measures for animal care, shelter and veterinary treatment, under lactation and gestation cycles used by dairy farmers. Under typical conditions for feeding and managing of lactating dairy cows, a total ration comprising *bm* silage and having a nutrient composition disclosed herein provides a significant increase in milk yield on a raw basis or fat-corrected basis, without adversely affecting general animal health, particularly live weight.

A total ration includes components such as fat, vitamins and minerals in proportions and within ranges that are known in the art. Maximum fiber digestibility occurs in a rumen environment suitable for growth of fiber-digesting organisms. Thus, the levels of other components are considered when formulating a total animal feed ration. For example, high levels of unsaturated fatty acids may depress NDF digestibility. In addition, the level of non-structural carbohydrate is adjusted to provide sufficient energy from starch fermentation without decreasing rumen pH to such an extent that the growth of fiber-digesting microbes is inhibited. Furthermore, the higher digestibility of *bm* corn silage may require that the ration be adjusted to achieve higher levels of non-protein nitrogen (NPN) in the rumen compared to rations using non-*bm* corn silage, because fiber-digesting microbes generally are more active when NPN is increased. Sufficient NPN can be provided by increasing the level and sources of SP in the ration, in combination with an increased forage/concentrate ratio.

Particle size of corn silage affects the rate and the efficiency of silage digestion in the rumen. A theoretical length of cut of about 0.95 cm to 1.27 cm is often used when chopping corn for silage. Because of the higher digestibility of *bm* corn silage, such silage may pass through the rumen more rapidly than desirable for efficient digestion and mat formation. Thus, a larger theoretical length of cut preferably is used when chopping *bm* corn, e.g., about 1.27 cm to about 2.54 cm, or about 1.91 cm to about 2.54 cm. Increased length of cut for *bm* corn silage contributes to increased scratch factor which stimulates cud-chewing and improves rumen buffering due to saliva formation.

From the foregoing it will be appreciated that the relative amounts of different components of the feed may be adjusted appropriately to allow maximum milk production. In particular, where necessary the level of CP should be increased to the upper level of the range given above, the feed should be formulated to maintain rumen pH around 6.2, such as in the range 5.5 to 7.0 for ruminants, particularly 5.5 to 6.5 for dairy cattle, for example by adjustment of the level of non-structural carbohydrate and factors which affect fiber digestibility should be adjusted appropriately, e.g. the levels of unsaturated fatty acids. Modification of each or all of the different components mentioned may be performed to enhance milk production. Alternatively, or additionally, the particle size of corn silage may be adjusted as mentioned above.

In one embodiment, a method according to the invention comprises administration of a biologically active somatotropin to a ruminant animal such as a dairy cow while feeding a ration comprising *bm* corn silage to the animal. Somatotropin refers to a peptide that has biological activity and chemical structure similar to those of somatotropin produced in the pituitary gland of an animal. Such somatotropin include, without limitation, natural somatotropins produced by pituitary somatotropic cells and somatotropins expressed by genetically transformed microorganisms such as *E. coli* or yeasts. Biologically active somatotropins may have an amino acid sequence identical to natural somatotropins or may be analogs having one or more alterations in amino acid sequence that provide enhanced biological activity or some other advantage. Illustrative embodiments of suitable somatotropins are described in, e.g., US Patents 4,693,973, 5,411,951, 5,037,806 and 5,086,041, all of which are incorporated herein by reference. A preferred somatotropin is bovine somatotropin for use with dairy cows.

Typically, somatotropin is administered parenterally, e.g., by intramuscular or subcutaneous injection, which delivers the peptide to the circulatory system. Suitable formulations for administering a somatotropin are known in the art. For example, somatotropin can be injected in a physiologically acceptable vehicle such as a wax and an oil. See, e.g., U.S. Patents 4,977,140, 5,474,980, 5,156,851 and 5,013,713, all of which are incorporated herein by reference. In some embodiments, a prolonged release formulation is used, such that a dose of somatotropin effective for enhancing milk production over a period of several days is released into the circulatory system with a single administration, e.g., at least 7 days, at least 14 days or at least 21 days.

A biologically active somatotropin is administered under conditions that provide an amount of peptide effective for enhancing milk production to a ruminant animal. Techniques and means are known in the art for determining appropriate doses to be supplied or provided to an individual animal. For example typically, amounts of somatotropin between about 5 and about 75 mg/cow/day are suitable for use in the invention. For example, parenteral administration of a single dose of about 300 mg of zinc-associated somatotropin can be sufficient to provide an increased amount of bovine somatotropin for a period of about 15 days. U.S. Patent 5,086,041, incorporated herein by reference.

A feed ration comprising *bm* corn silage is fed during a selected period to at least one lactating ruminant animal, e.g. dairy cow. Somatotropin is administered so that the production-enhancing effect of the peptide coincides with the feeding of the *bm* silage-containing ration. For example, a prolonged release formulation of somatotropin can be administered to a cow every 14 days over a 180 period. Concomitantly, a *bm* silage ration described above is fed daily to the cow over the same 180 day period. As an alternative, somatotropin can be administered daily and a *bm* silage ration can be fed daily. The somatotropin can be administered, e.g., by injection, either before or during the period in which the ration is being fed, provided that an effective amount of the peptide is delivered during the same period that the ration is being fed. The feeding period can be short, e.g., 4 days, or preferably longer, e.g., 10 days, 30 days or more.

A biologically active somatotropin may be administered to both primiparous and multiparous cows, either in early, mid or late lactation, e.g., about 63 to about 360 days postpartum. Somatotropin is particularly useful for increasing milk production during mid and late lactation; i.e., about 100 to about 360 days postpartum.

Feeding a dairy cattle feed ration comprising *bm* corn silage or *bm* corn silage results in surprising increases in milk production. An increase in milk production of at least about 1.8 kg/cow/day is often observed. A method of feeding a *bm* silage feed ration as disclosed herein provides significant utility to the producer because of such increases in milk production. Moreover, protein, lactose and solids-not-fat (SNF) components of milk are not altered significantly after feeding a *bm* silage total ration as disclosed herein.

Feeding a dairy cattle feed ration comprising *bm* corn silage and coextensively supplying an effective amount of a biologically active somatotropin also results in significant increases in milk production compared to the increase observed with somatotropin alone. For example, an increase in milk production of about 3 kg/cow/day can be observed over a 4 day period.

The invention will be further understood with reference to the following illustrative embodiments, which are purely exemplary and should not be taken as limiting the true scope of the present invention as described in the claims.

### Example 1

### Preparation of Silage from a Brown Midrib Hybrid

Inbred lines AR5152*bm3* and AR5751*bm3* are crossed (AR5152*bm3* X AR5751*bm*3) to make F₁ hybrid seed carrying the brown midrib genotype. Control hybrid seed is prepared by crossing the unconverted parental lines.

The control and *bm* hybrid seed is planted at a density of 24,900 kernels per acre and cultivated using a standard fertilization program. The corn is harvested 4 months after planting, using a forage harvester having a 0.95 cm theoretical length of cut (TLC) and a screen. The harvested material is placed in polyethylene bags (Ag Bags). If desired, the harvested material is treated with inoculant and/or preservative. The forage yield for *bm* corn silage is about 1 wet ton per acre less than the control hybrid corn.

Samples are collected from the polyethylene bags after 30 days of fermentation/storage and assayed for nutrient concentrations. Typical results are shown in Table 2. The control corn silage and the *bm* corn silage are similar in nutrient composition, except for fiber digestibility and NE_{L} concentration. The greater NE_{L} concentration is likely related to the *in vitro* digestibility of NDF, ADF, and hemicellulose.

**Table 2**

| **Nutrient composition of corn silages**^{**1**} | | |
|---|---|---|
| Nutrient | Control | BMR |
| Dry matter, % | 34.6 | 31.9 |
| Crude protein, % | 8.18 | 8.58 |
| Fat, % | 2.74 | 2.59 |
| ADF, % | 18.76 | 19.93 |
| NDF, % | 38.20 | 40.67 |
| Hemicellulose, % | 19.44 | 20.74 |
| NDF dig., % | 52.4 | 65.3 |
| ADF dig., % | 43.5 | 60.9 |
| Hemicellulose dig., % | 60.9 | 69.5 |
| Ash, % | 3.34 | 3.63 |
| NFC, % | 47.54 | 44 .53 |
| NE_{L}, Mcal/cwt. | 76.0 | 80.5 |
| Ca, % | .19 | .19 |
| P, % | .23 | .22 |
| Mg, % | .14 | .13 |
| K, % | 1.09 | 1.27 |
| Na, % | .01 | .09 |
| Salt, % | .22 | .13 |

| | | |
|---|---|---|
| ¹Nutrients are expressed on a dry matter basis | | |

### Example 2

### Formulation of a Dairy Cattle Feed Ration

The following experimental diets are developed to be fed as a total mixed ration (TMR). The following assumptions are used in ration formulation: the early lactation experimental dairy cow is estimated to weigh 636 kg and to produce 50 kg of milk per day with 3.30% fat, 3.10% milk protein and no body weight change. Dry matter intake (DMI) is predicted to be slightly less than 4.0% of body weight. The TMR is formulated to contain on a dry matter basis: 50% complete feed mix, 33% corn silage and 17% alfalfa haylage. The ingredient composition of the complete feed is given in Table 3 and the nutrient composition of the complete feed mix is given in Table 4.

**Table 3**

| **Ingredient Composition of Complete Feed**^{**1**} | |
|---|---|
| INGREDIENT | Composition (% by weight) |
| Corn, coarse ground | 38.7 |
| Wheat Middlings | 20.0 |
| Meat & Bone Meal (50% protein) | 1.5 |
| Hi Pro Soybean Meal (49% crude protein) | 1.9 |
| Feather Meal | 3.5 |
| Canola Meal | 20.0 |
| Animal fat | 2.3 |
| Minerals and Vitamins | 4.7 |
| Distillers grains | 7.4 |
| Total | 100.0 |

| | |
|---|---|
| ¹As fed basis. | |

**Table 4**

| **Nutrient Composition of Complete Feed** | |
|---|---|
| NUTRIENT | COMPOSITION¹ |
| Dry Matter | 88.0 |
| Crude Protein (CP) | 23.0 |
| Soluble Protein (SP as % of CP) | 21.0 |
| RUP (% of CP) | 45.0 |
| Fat | 5.0 |
| Net Energy of Lactation (NE_{L}) Mcal/kg | 92.0 |
| Acid Detergent Fiber (ADF) | 9.3 |
| Neutral Detergent Fiber (NDF) | 19.5 |
| Non-Fiber Carbohydrate (NFC) | 43.0 |
| Calcium | 1.40 |
| Phosphorus | 0.90 |
| Magnesium | 0.45 |
| Sulfur | 0.50 |
| Salt | 1.50 |

| | |
|---|---|
| ¹Percentages for nutrients given as % by weight on a dry matter basis | |

Brown midrib (BMR) or control corn silage (Example 2) are combined with complete feed mix and alfalfa haylage to form the rations, using each silage in an equal amount on a dry matter basis. Rations contain approximately 18.5% crude protein, 32% soluble protein and 40% rumen undegraded protein. Both rations are adjusted to a calculated pH of about 6.2 Both rations are balanced to meet or exceed mineral, vitamin and total bypass protein requirements.

### Example 3

### Milk Production Using BMR Silage in a Dairy Cattle Ration

20 lactating dairy cows are utilized in a randomized complete block design experiment, consisting of 5 primiparous cows and 5 multiparous cows fed control silage and 5 primiparous cows and 5 multiparous cows fed BMR silage. Cows are randomly assigned after calving. During the fifth week postpartum, the cows are abruptly switched to the appropriate feeding ration and continued on that ration from week 5 to week 17 postpartum. Primiparous cows are producing more than 25 kg/day of milk during the 4th week postpartum. Multiparous cows are producing greater than 29.5 kg/day of milk during the 4th week postpartum. All cows are disease free and otherwise healthy. Cows are fed ad libitum for the duration of the experiment, twice daily at 12 hour intervals, if possible. Cows are fed to allow for 5 to 10% feed refusal. The dietary ration and percentages of complete feed mix (Example 2), corn silage (Example 1) and alfalfa haylage on a dry matter basis are given in Table 5.

Milk production is measured at each milking and reported daily. Two samples of milk are taken weekly from each cow for composition analysis, including fat, protein, lactose, solids-not-fat and somatic cell counts. Body weights are recorded weekly after calving. Body condition scores are recorded at approximately weeks 4, 8, 12 and 16 postpartum. The same employee records condition scores at each measurement time. Body conditions scores are recorded according to the definitions indicated in Table 6.

**Table 5**

| **Dietary feeding guidelines to feed concentrate mix and forages** | | | | | |
|---|---|---|---|---|---|
| | Control ration | BMR ration | | Control ration | BMR ration |
| Ration Content | % of DM | % of DM | DM, % | % as Fed | % as Fed |
| Control Silage | 33.0 | - | 34.6 | 50.3 | - |
| BMR Silage | - | 33.0 | 31.9 | - | 52.4 |
| Complete Feed | 50.0 | 50.0 | 88.0 | 30.0 | 28.8 |
| Alfalfa Haylage | 17.0 | 17.0 | 45.6 | 19.7 | 18.9 |
| Total | 100.0 | 100.0 | | 100.0 | 100.0 |

The results indicate that cows fed BMR silage have a statistically significant increase in milk production when comparing unadjusted means or when comparing covariate adjusted means. The increase is from about 1.8 kg milk/cow/day to about 5.0 kg milk/cow/day. However, there is a statistically significant decrease in milkfat percentage in milk from cows fed the ration containing BMR silage.

**Table 6**

| **Instructions for Body Condition Scoring** | |
|---|---|
| Score | Definition |
| 1 | Loin area has limited flesh covering, is prominent and the ends of spinous processes are sharp to touch. Definite overhanging shelf effect is visible. Individual vertebrae of the hind quarters are prominent and distinct. Hooks and pin bones are notable. The area below the tail- head and between pin bones is severely depressed causing the bone structure of the area to appear extremely sharp. |
| | |
| 2 | Individual spinous processes are usually discernible but are not prominent. Ends of processes are sharp to touch, although they have greater flesh covering. The processes do not have a distinct overhanging shelf effect. Individual vertebrae of the hind quarters are not visually distinct but are readily distinguishable by palpitation. Hooks and pin bones are prominent, but the depression between them is less severe. The area below the tail-head and between the pin bones is depressed, but the bone structure is not devoid of flesh covering. |
| | |
| 3 | Spinous processes are discernible by applying slight pressure. Area over processes appears smooth and the overhanging shelf effect is not noticeable. Vertebrae of the hindquarters appear as a rounded ridge. Hooks and pin bones are rounded and smooth. The area between the pin bones and around the tail-head appear smooth without sign of fat deposition. |
| | |
| 4 | Individual spinous processes can be distinguished only by firm palpitation. Processes appear flat or rounded with no overhanging shelf effect. The ridge formed by the vertebrae of the hindquarters is rounded and smooth, flattening out as you move forward. Hooks are rounded, and the span between hooks is flat. The area around the tail- head and pin bones is rounded with evidence of fat deposition. |
| | |
| 5 | Bone structure of the vertebral column, spinous processes, hooks and pin bone regions is not visually apparent. Evidence of fat deposition is prominent. The tail head appears to be buried in fatty tissue. |

### Example 4

### Development of Corn Inbred Lines and Hybrids Having a Brown Midrib Phenotype

Line MED947 is a corn inbred that has the characteristics shown in Table 7. Line MED947 was crossed to B73*bm3* (stock no. 980392, obtained from the Department of Agronomy, Purdue University, West Lafayette, Indiana). After completing a conversion program, an inbred line designated as AR5252*bm3* was obtained. The backcrossing program is shown in Table 8.

Line MED154 is a corn inbred that has the characteristics shown in Table 9. Line MED154 was backcrossed to AR5151*bm3* as indicated in Table 10 to obtain line AR5251*bm3*. The *bm3* allele in AR5151*bm3* was obtained from A632*bm3* (Purdue University).

Line MED058 is a corn inbred that has the characteristics shown in Table 11. Line MED058 was backcrossed with line AR5654*bm3*. The *bm3* allele in AR5654*bm3* was derived from Mo17*bm*3 (Purdue University).

Line 7675 was crossed to Oh545*bm3* (stock no. 980390, Department of Agronomy, Purdue University) in a conversion program as shown in Table 8, resulting in an inbred line designated 7675*bm3*.

Line 7677 was crossed to Mo17*bm3* (stock no. 980394, Department of Agronomy, Purdue University) in a conversion program as shown in Table 8, resulting in an inbred line designated as 7677*bm3*.

Characteristics of the inbred lines AR5252*bm3*, 7675*bm3* and 7677*bm3* are shown in Table 12. Data on flowering for six inbred lines is shown in Table 13.

Line AR5252*bm3* was crossed as a female to 7675*bm3* and to 7677*bm3* to form F₁ hybrids 330666 and 330671, respectively. Characteristics of 330666 are shown in Table 14. Characteristics of 330671 are shown in Table 15.

Hybrid checks were prepared from crosses of AR5252 X 7675 and AR5252 X 7677. These F₁ hybrids do not exhibit the brown midrib phenotype.

Pairwise comparisons of forage yield, quality profiles and energy values for 330666 and unconverted control hybrid 118629 are shown in Table 16. The same comparisons are shown for 330671 and control hybrid X8311 in Table 17.

Lines AR5751*bm3*, AR5654*bm3*, AR5253*bm3*, AR5153*bm3* and AR5551*bm3* were developed by backcrossing programs similar to those described above. Each of these lines exhibits the brown midrib phenotype. Characteristics of these lines are shown in Tables 18-23.

**Table 7**

| **Description of Line MED947** | | | |
|---|---|---|---|
| COTYLEDON LEAF | | EAR CHARACTERISTICS | |
| ANTHOCYANIN | N/A | SILK COLOR | YELLOW |
| LENGTH | N/A | EAR LEAVES | ABSENT |
| | | HUSK | |
| PLANT AND STALK | | COVERAGE | COVERS TIP |
| PLANT HEIGHT | 7.50 | LOOSENESS | TIGHT |
| EAR HEIGHT | 3.00 | | |
| UNIFORMITY RATING | 8 | EAR ANGLE RATING | 6 |
| ANTHOCY. IN BRACE ROOTS | PRESENT | SHANK LENGTH | <4in. |
| ANTHOCY. IN NODES | ABSENT | EAR LENGTH | 6-9in. |
| DIAM. AT 2nd NODE | 0.00 | EAR SHAPE | CYLINDRICAL |
| ROOT RATING | N/A | UNIFORMITY | 8 |
| | | | |
| LEAVES | | KERNEL | |
| ANGLE | UPRIGHT | NO. ROWS | 16 |
| COLOR | MEDIUM GREEN | TYPE | SEMI DENT |
| TOTAL NUMBER | | SIZE | AVERAGE |
| NUMBER ABOVE EAR | 5-6 | BODY COLOR | YELLOW |
| LENGTH, EAR LEAF | 0 | CROWN COLOR | LIGHT YELLOW |
| MAX., WIDTH, EAR LEAF | 0.00 | | |
| ANTHOCYANIN, MARGIN | ABSENT | | |
| ANTHOCYANIN, SHEATH | ABSENT | COB | |
| PUBESCENCE, SHEATH | PRESENT | COLOR | PINK |
| PUBESCENCE, MARGINS | PRESENT | DIAMETER MIDPOINT | < 1in. |
| PLANT, TILLERING TASSEL | 0.00 | | |
| | | | |
| TASSEL | | INSECT RESISTANCE | |
| COMPACTNESS | COMPACT | ATTRACTIVE TO APHIDS | N/A |
| BRANCH ANGLE | >60 | | |
| NUMBER PRIMARY BRANCHES | 4-8 | DISEASE RESISTANCE RATINGS | |
| SECONDARY BRANCH | PRESENT | NORTHERN LEAF BLIGHT(R2) | 5B |
| LENGTH | N/A | SOUTHERN LEAF BLIGHT(R0) | 6B |
| SIZE RATING | 7 | NORTHERN COB LEAF SPOT(R3) | 6B |
| TASSEL EXTENSION PARTIALLY | ENCLOSED | STEWARTS BACTERIAL WILT | 6B |
| SHED IN BOOT? | NO | GREY LEAF SPOT | 8B |
| DIFFICULTY IN PULLING | AVERAGE | EYESPOT | 6B |
| NUMBER LEAVES PULLED | 0 | ANTHRACNOSE | 6B |
| ANTHER COLOR | YELLOW | (9=Resistant, 1=Susceptible) | |
| GLUME COLOR - TIP | RED | (Letter indicates confidence | |
| - BASE | LIGHT GREEN | level) | |
| - BAND | RED | | |
| POLLEN SHED DURATION | AVERAGE | | |
| POLLEN AMOUNT RATING | AVERAGE | | |

**Table 8**

| **Inbred Line Conversion to Brown MidRib** |
|---|
| AR5252*bm3* |
| (MED947XB73bm₃)MED947BC₆ |
| (MED947XB73bm₃)MED947BC₅S₁ |
| (MED947XB73bm₃)MED947BC₅ |
| (MED947XB73bm₃)MED947BC₄ |
| (MED947XB73bm₃)MED947BC₃S₁ |
| (MED947XB73bm₃)MED947BC₃ |
| (MED947XB73bm₃)MED947BC₂ |
| (MED947XB73bm₃)MED947BC₁S₁ |
| (MED947XB73bm₃)MED947BC₁ |
| (MED947XB73bm₃)F₁ |
| MED947XB73bm₃)F₀ |
| |

| 7675*bm3* |
|---|
| (7675xOh545bm₃)7675BC₆S₁ |
| (7675xOh545bm₃)7675BC₆ |
| (7675xOh545bm₃)7675BC₅ |
| (7675xOh545bm₃)7675BC₄ |
| (7675xOh545bm₃)7675BC₃S₁ |
| (7675xOh545bm₃)7675BC₃ |
| (7675xOh545bm₃)7675BC₂ |
| (7675xOh545bm₃)7675BC₁S₁ |
| (7675xOh545bm₃)7675BC₁ |
| (7675xOh545bm₃)7675F₁ |
| (7675xOh545bm₃)F₀ |
| |

| 7677*bm3* |
|---|
| |
| (7677XMo17bm₃)7677BC₅S₁ |
| (7677XMo17bm₃)7677BC₅ |
| (7677XMo17bm₃)7677BC₄ |
| (7677XMo17bm₃)7677BC₃S₁ |
| (7677XMo17bm₃)7677BC₃ |
| (7677XMo17bm₃)7677BC₂ |
| (7677XMo17bm₃)7677BC₁S₁ |
| (7677XMo17bm₃)7677BC₁ |
| (7677XMo17bm₃)F₁ |
| (7677XMo17bm₃)F₀ |

**Table 9**

| **Description of Line MED154** | | | |
|---|---|---|---|
| COTYLEDON LEAF | | EAR CHARACTERISTICS | |
| ANTHOCYANIN | N/A | SILK COLOR | RED |
| LENGTH | <2 | EAR LEAVES | MEDIUM |
| | | HUSK | |
| PLANT AND STALK | | COVERAGE | COVERS TIP |
| PLANT HEIGHT | 7.0 | LOOSENESS | LOOSE |
| EAR HEIGHT | 2.0 | | |
| UNIFORMITY RATING | 7 | EAR ANGLE RATING | 6 |
| ANTHOCY. IN BRACE ROOTS | PRESENT | SHANK LENGTH | 4-12 |
| ANTHOCY. IN NODES | ABSENT | EAR LENGTH | 6-9 |
| DIAM. AT 2nd NODE | 1.0 | EAR SHAPE | CONICAL |
| ROOT RATING | AVERAGE | UNIFORMITY | 7 |
| | | | |
| LEAVES | | KERNEL | |
| ANGLE | VERY | NO. ROWS | 16 |
| COLOR | MEDIUM | TYPE | DENT |
| TOTAL NUMBER | 10-15 | SIZE | AVERAGE |
| NUMBER ABOVE EAR | 5-6 | BODY COLOR | ORANGE |
| LENGTH, EAR LEAF | 34 | CROWN COLOR | YELLOW |
| MAX., WIDTH, EAR LEAF | 4.50 | | |
| ANTHOCYANIN, MARGIN | PRESENT | | |
| ANTHOCYANIN, SHEATH | ABSENT | COB | |
| PUBESCENCE, SHEATH | ABSENT | COLOR | WHITE |
| PUBESCENCE, MARGINS | PRESENT | DIAMETER MIDPOINT | 1-2 |
| PLANT, TILLERING TASSEL | 0.00 | | |
| | | | |
| TASSEL | | INSECT RESISTANCE | |
| COMPACTNESS | AVERAGE | ATTRACTIVE TO APHIDS | YES |
| BRANCH ANGLE | 30-60 | | |
| NUMBER PRIMARY BRANCHES | <4 | | |
| SECONDARY BRANCH | ABSENT | | |
| LENGTH | 10-14 | | |
| SIZE RATING | 2 | | |
| TASSEL EXTENSION | PARTIALLY ENCLOSED | | |
| TASSEL FERTILITY | 0 | | |
| SHED IN BOOT? | NO | | |
| DIFFICULTY IN PULLING | AVERAGE | | |
| NUMBER LEAVES PULLED | 1 | | |
| ANTHER COLOR | PINK | | |
| GLUME COLOR - TIP | LIGHT GREEN | | |
| - BASE | RED | | |
| - BAND | DARK GREEN | | |
| POLLEN SHED DURATION | SHORT | | |
| POLLEN AMOUNT RATING | LONG | | |

**Table 10**

| **Inbred Line Conversion to Brown Midrib** |
|---|
| AR5251*bm3* |
| |
| (MED154XAR5151bm₃)C₅ |
| (MED154XAR5151bm₃)C₄ |
| (MED154XAR5151bm₃)C₃S₁ |
| (MED154XAR5151bm₃)C₃ |
| (MED154XAR5151bm₃)C₂ |
| (MED154XAR5151bm₃)C₁S₁ |
| (MED154XAR5151bm₃)C₁ |
| (MED154XAR5151bm₃)F₁ |
| (MED154XAR5151bm₃)F₀ |
| |

| AR5651*bm3* |
|---|
| |
| (MED058XAR5654bm₃)C₅S₁ |
| (MED058XAR5654bm₃)C₅ |
| (MED058XAR5654bm₃)C₄ |
| (MED058XAR5654bm₃)C₃ |
| (MED058XAR5654bm₃)C₂ |
| (MED058XAR5654bm₃)C₁S₁ |
| (MED058XAR5654bm₃)C₁ |
| (MED058XAR5654bm₃)F₁ |
| (MED058XAR5654bm₃)F₀ |

**Table 11**

| **Description of Line MED058** | | | |
|---|---|---|---|
| COTYLEDON LEAF | | EAR CHARACTERISTICS | |
| ANTHOCYANIN | N/A | SILK COLOR | YELLOW |
| LENGTH | <2 | EAR LEAVES | ABSENT |
| | | HUSK | |
| PLANT AND STALK | | COVERAGE | COVERS TIP |
| PLANT HEIGHT | 6.5 | LOOSENESS | AVERAGE |
| EAR HEIGHT | 3.0 | | |
| UNIFORMITY RATING | 7 | EAR ANGLE RATING | 5 |
| ANTHOCY. IN BRACE ROOTS | PRESENT | SHANK LENGTH | <4 |
| ANTHOCY. IN NODES | ABSENT | EAR LENGTH | 6-9 |
| DIAM. AT 2nd NODE | 1.0 | EAR SHAPE | CYLINDRICAL |
| ROOT RATING | AVERAGE | UNIFORMITY | 9 |
| | | | |
| LEAVES | | KERNEL | |
| ANGLE | UPRIGHT | NO. ROWS | 12 |
| COLOR | LIGHT GREEN | TYPE | DENT |
| TOTAL NUMBER | 10-15 | SIZE | AVERAGE |
| NUMBER ABOVE EAR | >6 | BODY COLOR | ORANGE |
| LENGTH, EAR LEAF | 21 | CROWN COLOR | YELLOW |
| MAX., WIDTH, EAR LEAF | 3.00 | | |
| ANTHOCYANIN, MARGIN | PRESENT | | |
| ANTHOCYANIN, SHEATH | ABSENT | COB | |
| PUBESCENCE, SHEATH | ABSENT | COLOR | WHITE |
| PUBESCENCE, MARGINS | ABSENT | DIAMETER MIDPOINT | <1 |
| PLANT, TILLERING TASSEL | 0.00 | | |
| | | | |
| TASSEL | | INSECT RESISTANCE | |
| COMPACTNESS | LOOSE | ATTRACTIVE TO APHIDS | NO |
| BRANCH ANGLE | >60 | | |
| NUMBER PRIMARY BRANCHES | 4-6 | | |
| SECONDARY BRANCH | ABSENT | | |
| LENGTH | >14 | | |
| SIZE RATING | 5 | | |
| TASSEL EXTENSION | PARTIALLY ENCLOSED | | |
| TASSEL FERTILITY | 0 | | |
| SHED IN BOOT? | NO | | |
| DIFFICULTY IN PULLING | EASY | | |
| NUMBER LEAVES PULLED | 2 | | |
| ANTHER COLOR | YELLOW | | |
| GLUME COLOR - TIP | LIGHT GREEN | | |
| - BASE | LIGHT GREEN | | |
| - BAND | DARK GREEN | | |
| POLLEN SHED DURATION | AVERAGE | | |
| POLLEN AMOUNT RATING | LIGHT | | |

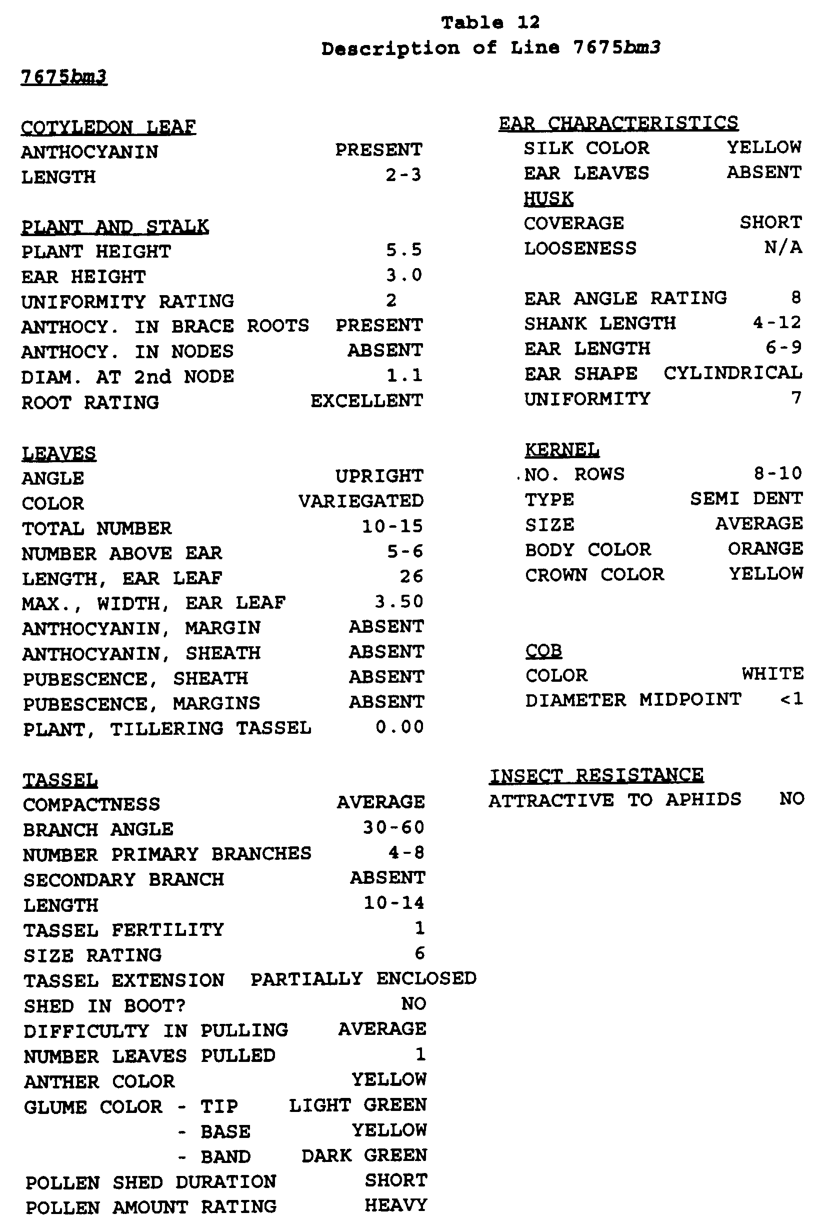

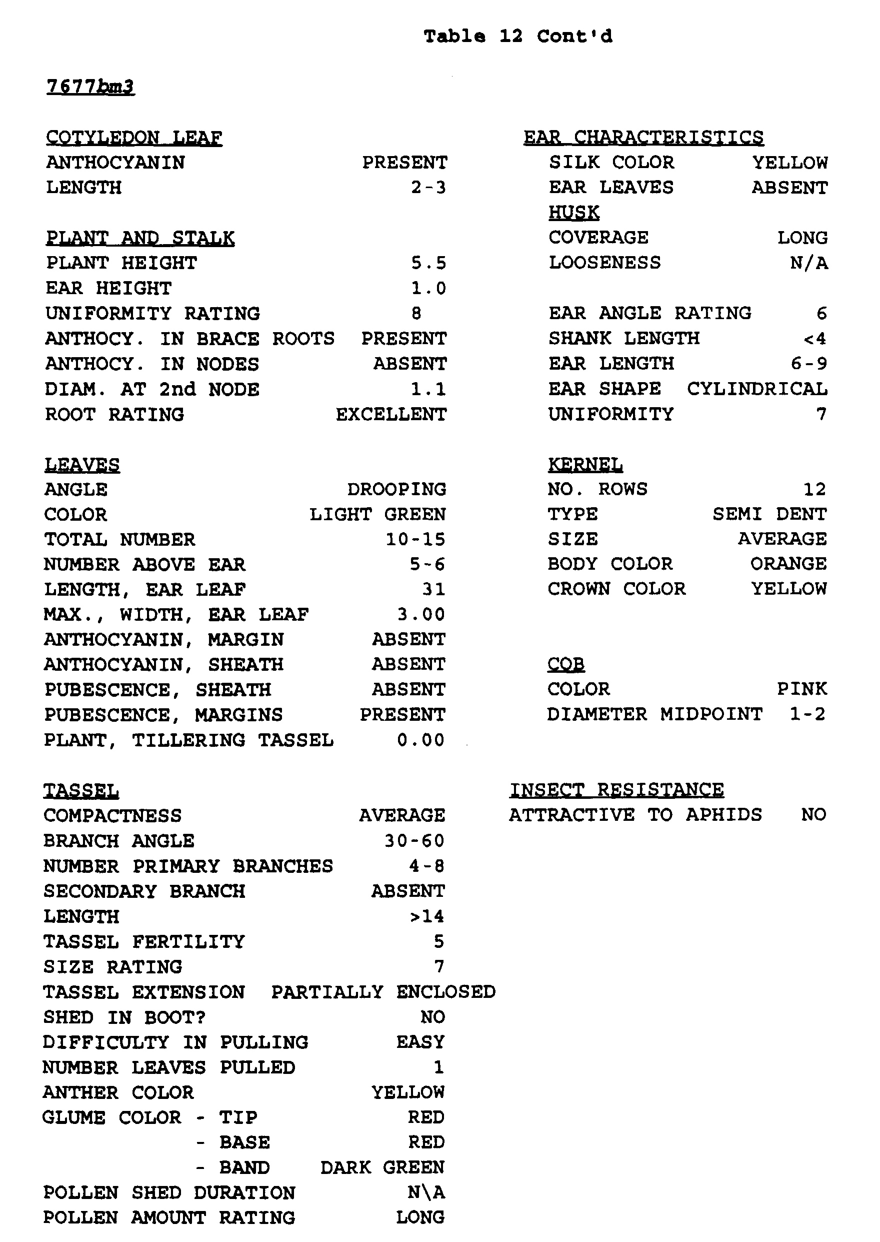

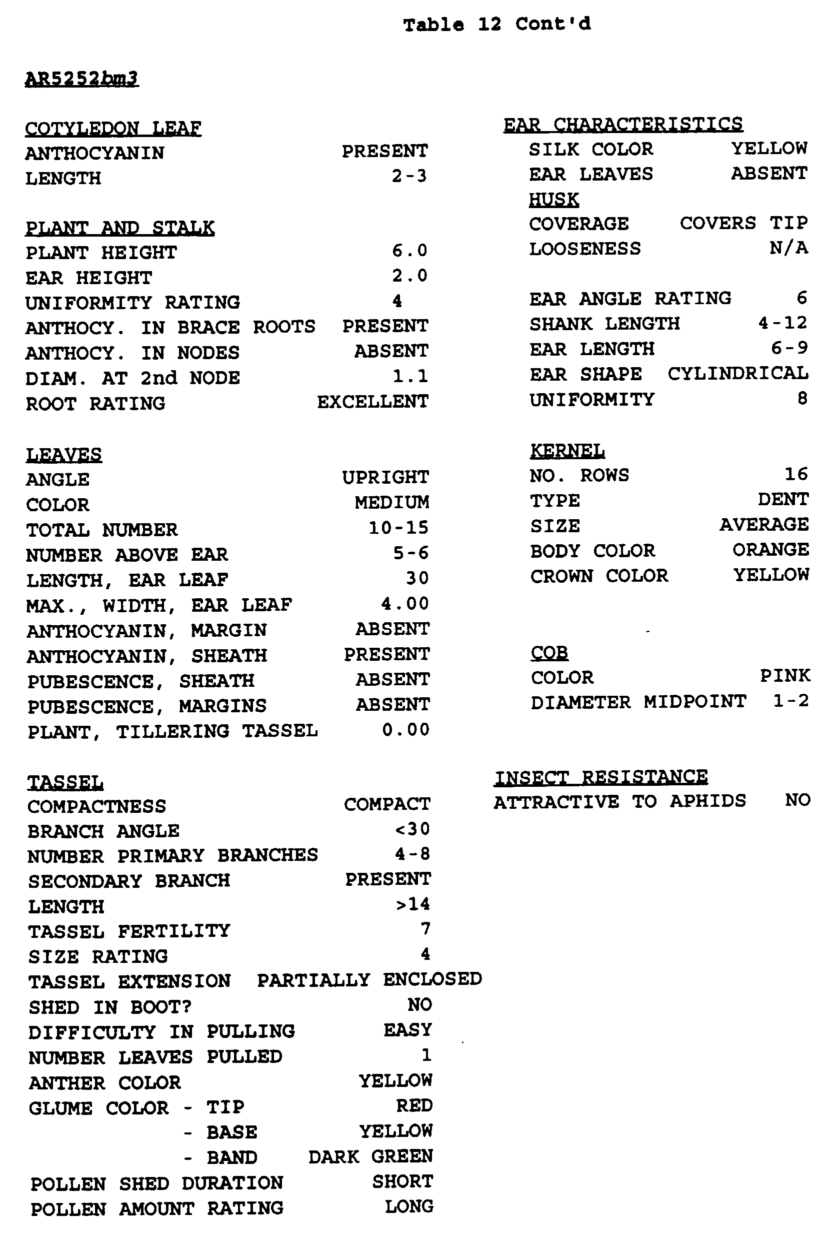

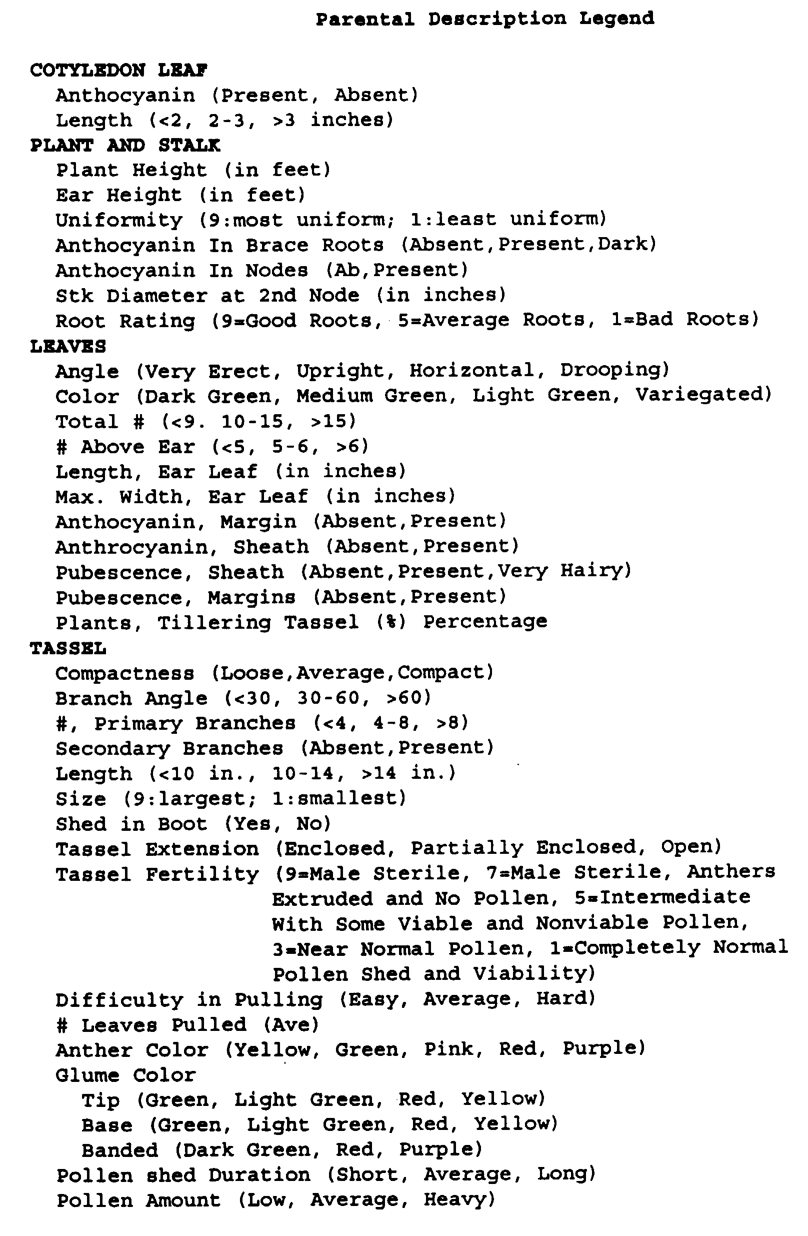

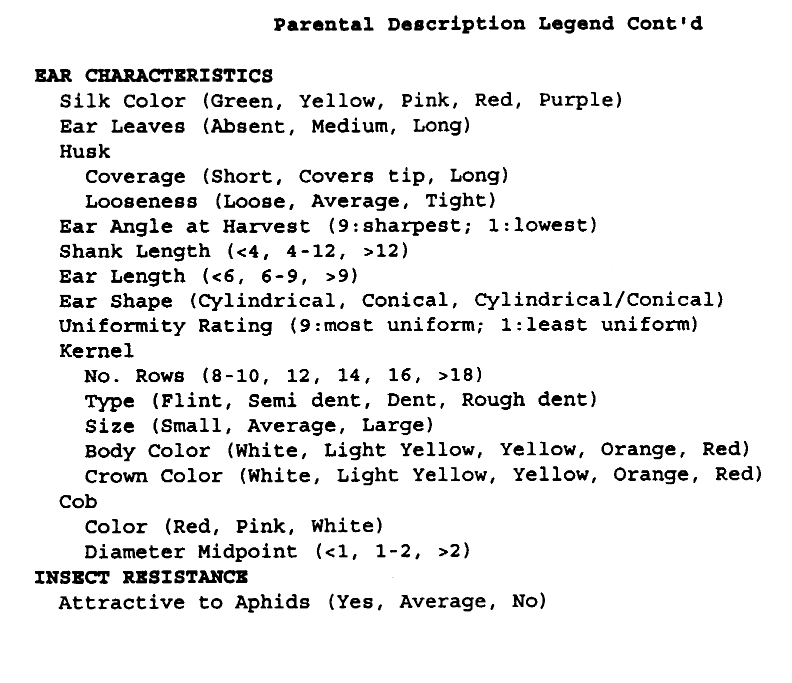

**Table 13**

| **Flowering Characteristics of *bm* Inbred Lines** | | | | | |
|---|---|---|---|---|---|
| 7675*bm3* | | AR5252*bm3* | | 7677*bm3* | |
| Slk Heat 1 | 1,564 | Slk Heat 1 | 1,534 | Slk Heat 1 | 1,683 |
| Slk Heat M | 1,587 | Slk Heat M | 1,607 | Slk Heat M | 1,738 |
| Slk Heat F | 1,633 | Slk Heat F | 1,587 | Slk Heat F | 1,794 |
| Slk Days 1 | 86 | Slk Days 1 | 85 | Slk Days 1 | 91 |
| Slk Days M | 87 | Slk Days M | 88 | Slk Days M | 93 |
| Slk Days F | 89 | Slk Days F | 87 | Slk Days F | 95 |
| Pol Heat 1 | 1,534 | Pol Heat 1 | 1,505 | Pol Heat 1 | 1,419 |
| Pol Heat M | 1,587 | Pol Heat M | 1,534 | Pol Heat M | 1,587 |
| Pol Heat F | 1,607 | Pol Heat F | 1,587 | Pol Heat F | 1,633 |
| Pol Days 1 | 85 | Pol Days 1 | 84 | Pol Days 1 | 80 |
| Pol Days M | 87 | Pol Days M | 85 | Pol Days M | 87 |
| Pol Days F | 88 | Pol Days F | 87 | Pol Days F | 89 |

| AR5651*bm3* | | AR5251*bm3* | | | |
|---|---|---|---|---|---|
| Slk Heat 1 | 1,387 | Slk Heat 1 | 1,552 | | |
| Slk Heat M | 1,437 | Slk Heat M | 1,582 | | |
| Slk Heat F | 1,479 | Slk Heat F | 1,656 | | |
| Slk Days 1 | 83 | Slk Days 1 | 91 | | |
| Slk Days M | 85 | Slk Days M | 93 | | |
| Slk Days F | 87 | Slk Days F | 97 | | |
| Pol Heat 1 | 1,304 | Pol Heat 1 | 1,539 | | |
| Pol Heat M | 1,387 | Pol Heat M | 1,552 | | |
| Pol Heat F | 1,459 | Pol Heat F | 1,596 | | |
| Pol Days 1 | 79 | Pol Days 1 | 90 | | |
| Pol Days M | 83 | Pol Days M | 91 | | |
| Pol Days F | 86 | Pol Days F | 94 | | |

**Table 14**

| **Trait Summary for 330666** | | | |
|---|---|---|---|
| | RATING | SCALE | DEFINITION |
| | | | |
| | | | |

| Trait: | | | |
|---|---|---|---|
| Yield for Maturity (9-1) | 9 | 9-1 | 9=GOOD 1=POOR |
| Stalk Strength (9-1) | Acceptable | 9-1 | 9=GOOD 1=POOR |
| Root Strength-Summer (9-1) | 9 | 9-1 | 9=GOOD 1=POOR |
| Root Strength-Fall (9-1) | NA | 9-1 | 9=GOOD 1=POOR |
| Testweight (9-1) | NA | 9-1 | 9=GOOD 1=POOR |
| Drydown (9-1) | NA | 9-1 | 9=GOOD 1=POOR |
| Ear Retention (9-1) | 9 | 9-1 | 9=GOOD 1=POOR |
| Early Vigor (9-1) | 5 | 9-1 | 9=GOOD 1=POOR |
| Seedling Purple Color (9-1) | 4 | 9-1 | 9=NO PURPLE 1=DARK PURPLE |
| Greensnap Potential (9-1) | 9 | 9-1 | 9=LOW 1=HIGH |
| Drought Stress (9-1) | 7 | 9-1 | 9=GOOD 1=POOR |
| Stay Green (9-1) | 8 | 9-1 | 9=GOOD 1=POOR |
| Plant Health (9-1) | 7 | 9-1 | 9=GOOD 1=POOR |
| Recommended Population (H M L) | H | H,M,L | HIGH, MEDIUM, LOW |
| Plant Height (S MS M MT T) | 8.0 | S,MS,M,MT,T | SHORT/MSHORT/MODERATE/MTALL/TA |
| Ear Height (S MS M MT T) | 3.5 | S,MS,M,MT,T | SHORT/MSHORT/MODERATE/MTALL/TA |
| | | | |
| | | | |
| Cargill RM (# Days) | 115 (NA) | DAYS | # DAYS |
| Flowering Date (E M L) | M | E,M,L | EARLY,MEDIUM,LATE |
| GDU's to Mid-Silk (#) | 1464 | # | # UNITS |
| GDU's to Black Layer (#) | 2594 | # | # UNITS |
| | | | |
| | | | |
| Plant Color (L M D) | M | L,M,D | LIGHT,MEDIUM,DARK |
| Leaf Angle (E U H D) | U | E,U,H,D | ERECT/UPRIGHT/HORIZONTAL.DROOPY |
| Leaf Width (W M N) | M | W,M,N | WIDE,MEDIUM,NARROW |
| Tillers (9-1) | 8 | 9-1 | 9=NONE 1=MANY |
| Shank Length (9-1) | 5 | 9-1 | 9=SHORT 1=LONG |
| Husk Coverage (9-1) | 8 | 9-1 | 9=GOOD 1=POOR |
| Ear Tip Fill (9-1) | 6 | 9-1 | 9=GOOD 1=POOR |
| Ear Length (L M S) | M | L,M,S | LONG,MEDIUM,SHORT |
| Ear Girth (G M S) | M | G,M,S | GIRTHY,MEDIUM,SLENDER |
| Ear Flex (9-1) | NA | 9-1 | 9=GOOD 1=POOR |
| Kernel Rows (# Range) | 14-16 | # | # Range |
| Kernel Depth (S M D) | D | S,M,D | SHALLOW,MODERATE,DEEP |
| Cob Color (R P W) | P | R,P,W | RED,PINK,WHITE |
| Kernel Color (W Y G B O R) | Y | W,Y,G,B,O,R | WHITE,YLLW,GLD,BRNZ,ORNG,RED |
| Kernel Texture (F SF SD D) | SD | F,SF,SD,D | FLINT,SEMIFLINT,SEMIDENT,DENT |
| | | | |

**Table 15**

| **Trait Summary for 330671** | | | |
|---|---|---|---|
| | RATING | SCALE | DEFINITION |
| Trait: | | | |
| Yield for Maturity (9-1) | 7 | 9-1 | 9=GOOD 1=POOR |
| Stalk Strength (9-1) | Acceptable | 9-1 | 9=GOOD 1=POOR |
| Root Strength-Summer (9-1) | 9 | 9-1 | 9=GOOD 1=POOR |
| Root Strength-Fall (9-1) | NA | 9-1 | 9=GOOD 1=POOR |
| Testweight (9-1) | NA | 9-1 | 9=GOOD 1=POOR |
| Drydown (9-1) | NA | 9-1 | 9=GOOD 1=POOR |
| Ear Retention (9-1) | 9 | 9-1 | 9=GOOD 1=POOR |
| Early Vigor (9-1) | 5 | 9-1 | 9=GOOD 1=POOR |
| Seedling Purple Color (9-1) | | 9-1 | 9=NO PURPLE 1=DARK PURPLE |
| Greensnap Potential (9-1) | 8 | 9-1 | 9=LOW 1=HIGH |
| Drought Stress (9-1) | 8 | 9-1 | 9=GOOD 1=POOR |
| Stay Green (9-1) | 8 | 9-1 | 9=GOOD 1=POOR |
| Plant Health (9-1) | 8 | 9-1 | 9=GOOD 1=POOR |
| Recommended Population (H M L) | H | H,M,L | HIGH, MEDIUM, LOW |
| Plant Height (S MS M MT T) | 8.5 | S,MS,M,MT,T | SHORT/MSHORT/MODERATE/MTALL/TA |
| Ear Height (S MS M MT T) | 3.0 | S,MS,M,MT,T | SHORT/MSHORT/MODERATE/MTALL/TA |
| | | | |
| | | | |
| Cargill RM (# Days) | | DAYS | # DAYS |
| Flowering Date (E M L) | M | E,M,L | EARLY,MEDIUM,LATE |
| GDU's to Mid-Silk (#) | 1529 | # | # UNITS |
| GDU's to Black Layer (#) | NA | # | # UNITS |
| | | | |
| | | | |
| Plant Color (L M D) | | L,M,D | LIGHT,MEDIUM,DARK |
| Leaf Angle (E U H D) | | E,U,H,D | ERECT/UPRIGHT/HORIZONTAL.DROOPY |
| Leaf Width (W M N) | | W,M,N | WIDE,MEDIUM,NARROW |
| Tillers (9-1) | | 9-1 | 9=NONE 1=MANY |
| Shank Length (9-1) | | 9-1 | 9=SHORT 1=LONG |
| Husk Coverage (9-1) | | 9-1 | 9=GOOD 1=POOR |
| Ear Tip Fill (9-1) | | 9-1 | 9=GOOD 1=POOR |
| Ear Length (L M S) | | L,M,S | LONG,MEDIUM,SHORT |
| Ear Girth (G M S) | | G,M,S | GIRTHY,MEDIUM,SLENDER |
| Ear Flex (9-1) | NA | 9-1 | 9=GOOD 1=POOR |
| Kernel Rows (# Range) | | # | # Range |
| Kernel Depth (S M D) | | S,M,D | SHALLOW,MODERATE,DEEP |
| Cob Color (R P W) | P | R,P,W | RED,PINK,WHITE |
| Kernel Color (W Y G B O R) | | W,Y,G,B,O,R | WHITE,YLLW,GLD,BRNZ,ORNG,RED |
| Kernel Texture (F SF SD D) | | F,SF,SD,D | FLINT,SEMIFLINT,SEMIDENT,DENT |
| | | | |

**Table 16**

| **Pairwise Comparison of 330666 and 118629**^{**a**} | | | | |
|---|---|---|---|---|
| Traits | 330666 | 118629 | Range | #Loc. |
| **Plant Features** | | | | |
| Plant Hgt | 45.54 | 47.18 | -1.64 | 5 |
| Ear Hgt | 18.80 | 19.80 | -1.00 | 5 |

| **Yield Profile**^{**b**} | | | | |
|---|---|---|---|---|
| 70% Tons/A | 25.00 | 27.49 | -2.49 | 6 |
| DM Tons/A | 7.50 | 8.25 | -0.75 | 6 |
| DDM Tons/A | 5.90 | 6.07 | -0.17 | 6 |

| **Quality Profile**^{**c**} | | | | |
|---|---|---|---|---|
| % ADF | 23.18 | 24.83 | -1.65 | 6 |
| % NDF | 43.56 | 45.08 | -1.52 | 6 |
| % ASH | 4.62 | 4.51 | 0.11 | 6 |
| % CP | 8.92 | 8.29 | 0.63 | 6 |
| % IVTD | 78.75 | 73.26 | 5.49 | 6 |
| % IVCWD | 50.99 | 40.64 | 10.35 | 6 |
| % WP Mst | 70.83 | 66.54 | 4.29 | 6 |
| % WP DM | 29.17 | 33.46 | -4.29 | 6 |

| **Energy Values**^{**d**} | | | | |
|---|---|---|---|---|
| TDN | 67.97 | 66.28 | 1.69 | 6 |
| NEL (Mcal/LB) | 0.69 | 0.67 | 0.02 | 6 |
| NFC% | 39.80 | 39.02 | 0.78 | 6 |
| Lbs CP/A | 1,330.26 | 1,355.60 | -25.34 | 6 |
| Lbs TDN/A | 10,223.68 | 11,006.10 | -782.42 | 6 |
| Lbs NEL/A | 10,295.38 | 11,028.58 | -733.20 | 6 |
| Lbs DDM/Ton | 1,574.90 | 1,465.23 | 109.67 | 6 |
| LBS CP/Ton | 178.40 | 165.87 | 12.53 | 6 |

| | | | | |
|---|---|---|---|---|
| ^{a}Summary over two years | | | | |
| ^{b}DM=Dry Matter; DDM=Digestible Dry Matter; Tons/A=Tons per acre | | | | |
| ^{c}ADF=Acid Detergent Fiber; WP Mst=whole plant moisture; UPDM=Whole Plant Dry Matter | | | | |
| ^{d}TDN=Total Digestible Nutrients in % by weight (dry basis); NEL=Net Energy of Lactation in Mcal per pound. | | | | |

**Table 17**

| **Pairwise Comparison of 330671 and X8311** | | | | |
|---|---|---|---|---|
| Traits | 330671 | X8311 | Range | #Loc. |
| **Plant Features** | | | | |
| Plant Hgt | 45.28 | 49.38 | -4.10 | 5 |
| Ear Hgt | 19.06 | 21.42 | -2.36 | 5 |

| **Yield Profile** | | | | |
|---|---|---|---|---|
| 70% Tons/A | 27.00 | 30.23 | -3.23 | 6 |
| DM Tons/A | 8.10 | 9.07 | -0.97 | 6 |
| DDM Tons/A | 6.22 | 6.50 | -0.28 | 6 |

| **Quality Profile** | | | | |
|---|---|---|---|---|
| % ADF | 23.29 | 25.39 | -2.10 | 6 |
| % NDF | 44.96 | 46.97 | -2.01 | 6 |
| % ASH | 4.59 | 4.32 | 0.27 | 6 |
| % CP | 8.49 | 8.12 | 0.37 | 6 |
| % IVTD | 76.65 | 71.79 | 4.86 | 6 |
| % IVCWD | 47.88 | 39.89 | 7.99 | 6 |
| % WP Mst | 69.93 | 68.17 | 1.76 | 6 |
| % WP DM | 30.07 | 31.83 | -1.76 | 6 |

| **Energy Values** | | | | |
|---|---|---|---|---|
| TDN | 67.82 | 65.68 | 2.14 | 6 |
| NEL (Mcal/LB) | 0.68 | 0.66 | 0.02 | 6 |
| NFC% | 38.85 | 37.50 | 1.35 | 6 |
| Lbs CP/A | 1,354.67 | 1,474.91 | -120.24 | 6 |
| Lbs TDN/A | 11,019.01 | 11,942.15 | -923.14 | 6 |
| Lbs NEL/A | 11,097.71 | 11,935.90 | -838.19 | 6 |
| Lbs DDM/Ton | 1,533.00 | 1,435.80 | 97.20 | 6 |
| LBS CP/Ton | 169.87 | 162.33 | 7.54 | 6 |
| *Summary over two years of data at 6 locations | | | | |

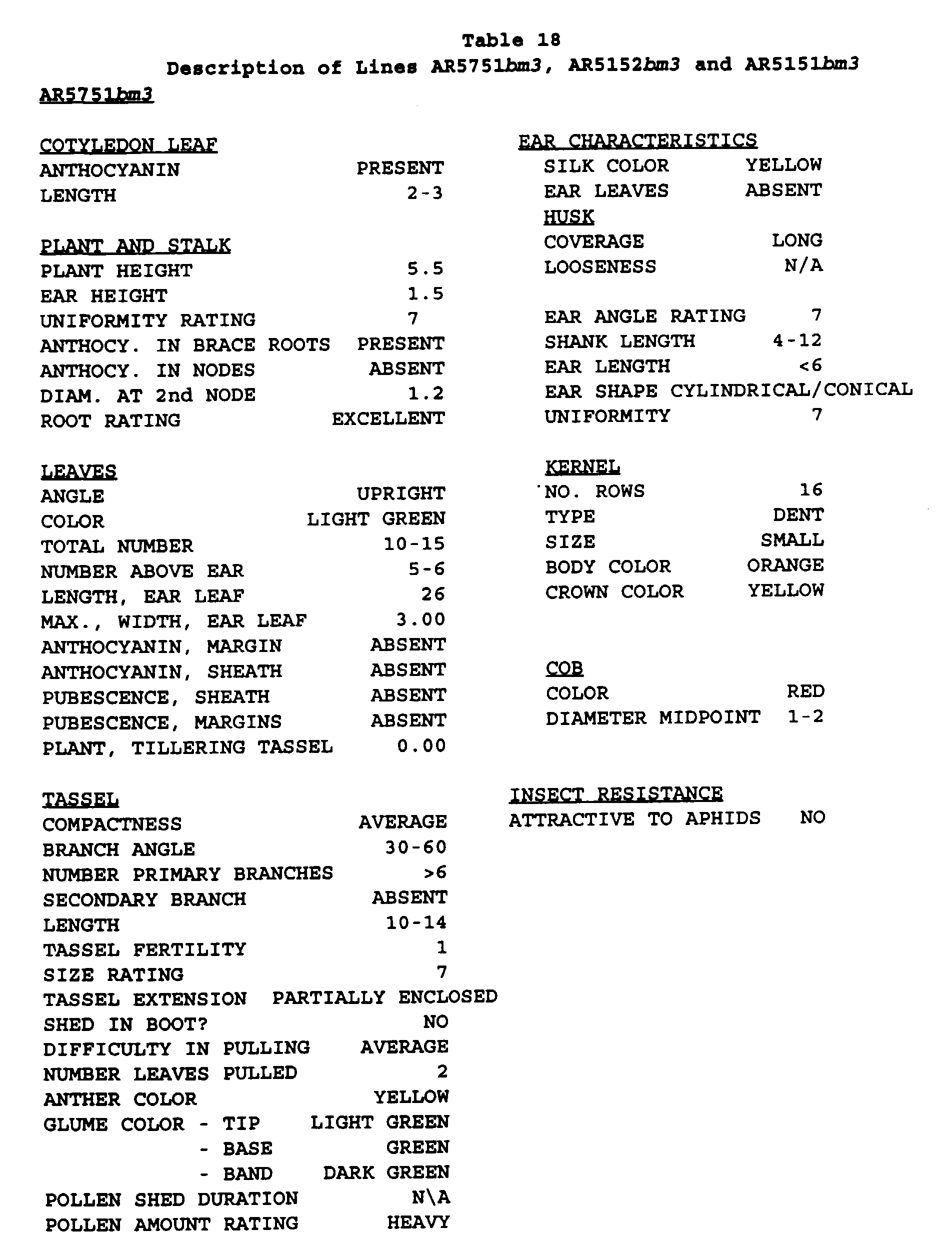

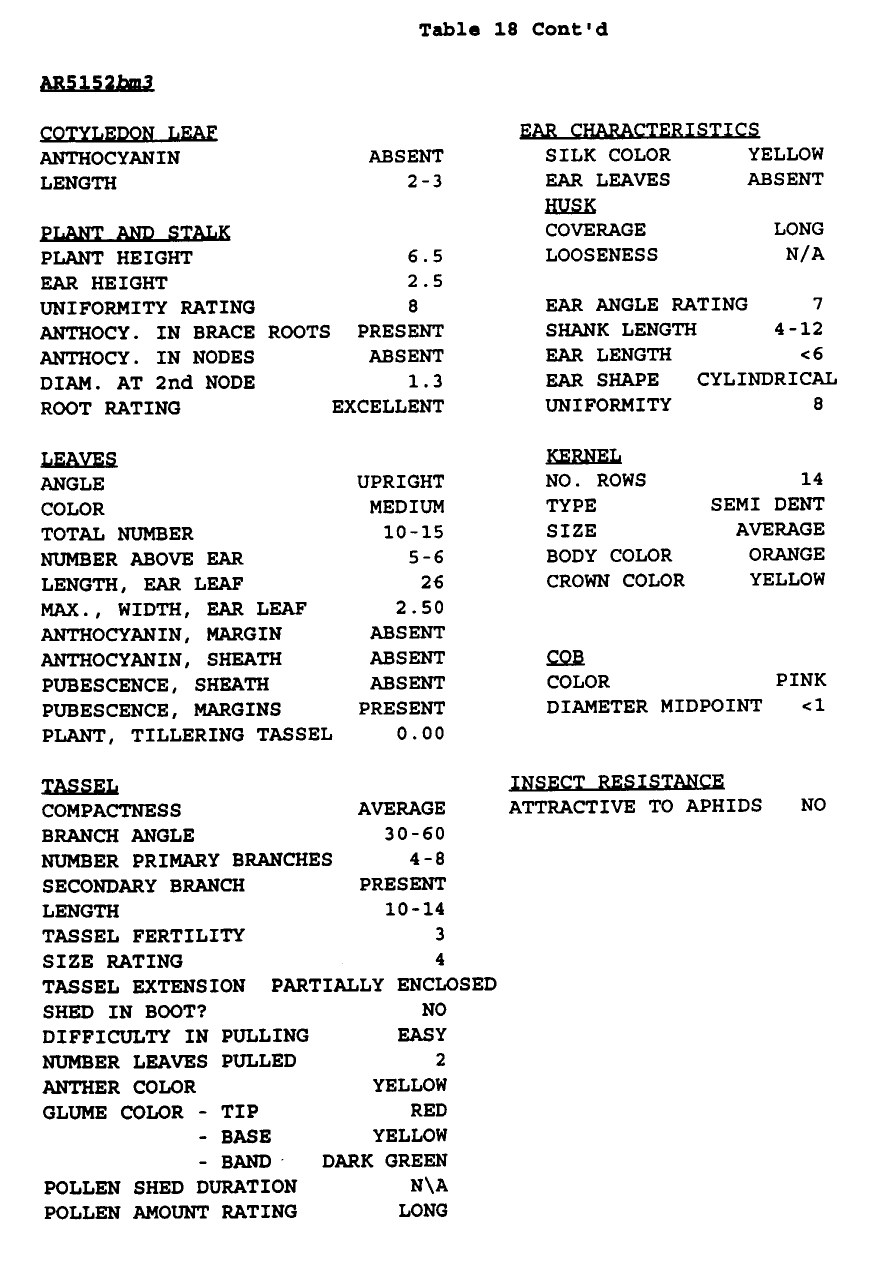

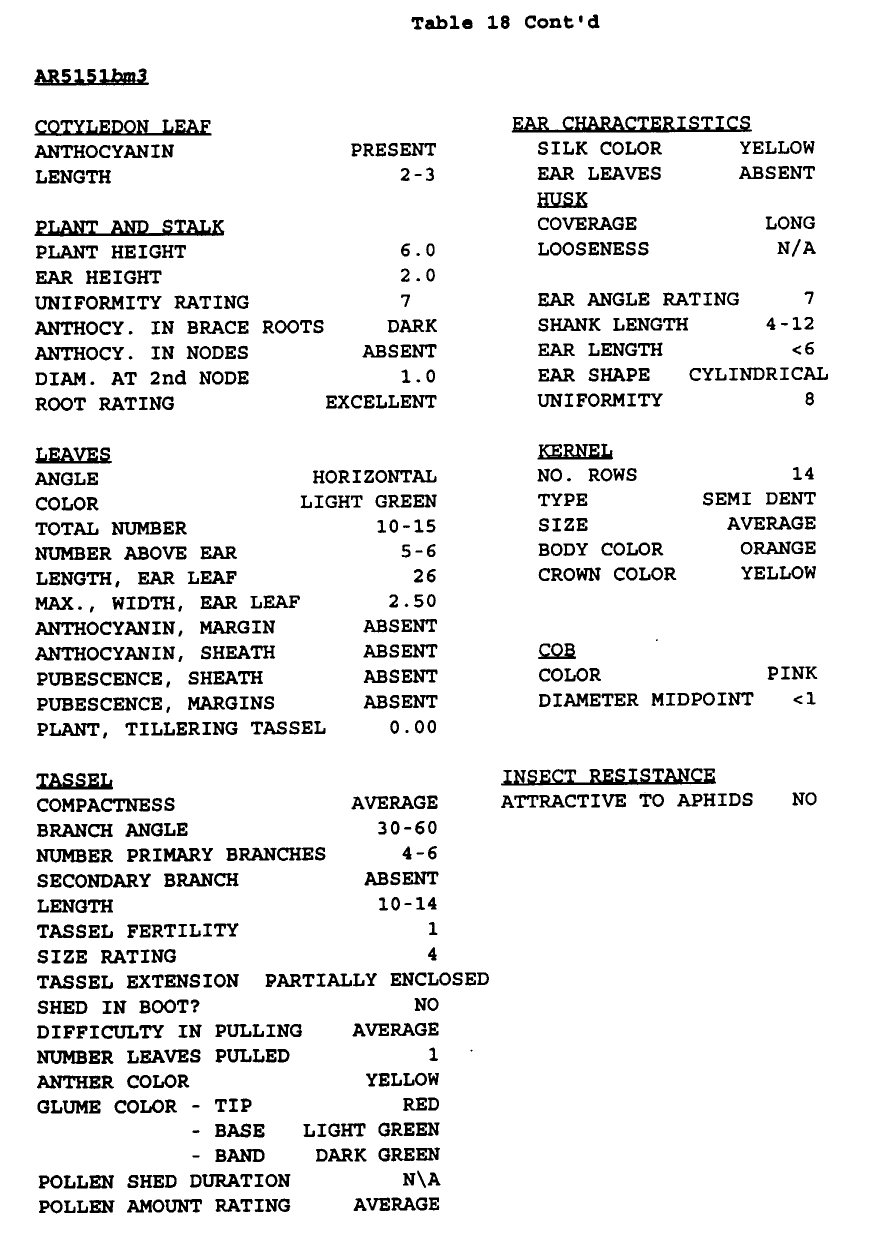

**Table 19**

| AR5751BM3 | |
|---|---|
| Slk Heat 1 | 1,441 |
| Slk Heat M | 1,463 |
| Slk Heat F | 1,505 |
| Slk Days 1 | 81 |
| Slk Days M | 82 |
| Slk Days F | 84 |
| Pol Heat 1 | 1,463 |
| Pol Heat M | 1,485 |
| Pol Heat F | 1,505 |
| Pol Days 1 | 82 |
| Pol Days M | 83 |
| Pol Days F | 84 |

| AR5152BM3 | |
|---|---|
| Slk Heat 1 | 1,587 |
| Slk Heat M | 1,607 |
| Slk Heat F | 1,633 |
| Slk Days 1 | 87 |
| Slk Days M | 88 |
| Slk Days F | 89 |
| Pol Heat 1 | 1,534 |
| Pol Heat M | 1,564 |
| Pol Heat F | 1,587 |
| Pol Days 1 | 85 |
| Pol Days M | 86 |
| Pol Days F | 87 |

| AR5151BM3 | |
|---|---|
| Slk Heat 1 | 1,256 |
| Slk Heat M | 1,300 |
| Slk Heat F | 1,345 |
| Slk Days 1 | 73 |
| Slk Days M | 75 |
| Slk Days F | 77 |
| Pol Heat 1 | 1,175 |
| Pol Heat M | 1,231 |
| Pol Heat F | 1,345 |
| Pol Days 1 | 70 |
| Pol Days M | 72 |
| Pol Days F | 77 |

**Table 20**

| **Description of Line AR5654*bm3*** | | | |
|---|---|---|---|
| **AR5654*bm3*** | | | |
| COTYLEDON LEAF | | EAR CHARACTERISTICS | |
| ANTHOCYANIN | PRESENT | SILK COLOR | PINK |
| LENGTH | 2-3 | EAR LEAVES | ABSENT |
| | | HUSK | |
| PLANT AND STALK | | COVERAGE | COVERS TIP |
| PLANT HEIGHT | 6.0 | LOOSENESS | N/A |
| EAR HEIGHT | 1.5 | | |
| UNIFORMITY RATING | 7 | EAR ANGLE RATING | 6 |
| ANTHOCY. IN BRACE ROOTS | PRESENT | SHANK LENGTH | 4-12 |
| ANTHOCY. IN NODES | ABSENT | EAR LENGTH | <6 |
| DIAM. AT 2nd NODE | 1.1 | EAR SHAPE | CYLINDRICAL |
| ROOT RATING | EXCELLENT | UNIFORMITY | 7 |
| | | | |
| LEAVES | | KERNEL | |
| ANGLE | HORIZONTAL | NO. ROWS | 14 |
| COLOR | MEDIUM | TYPE | FLINT |
| TOTAL NUMBER | 10-15 | SIZE | SMALL |
| NUMBER ABOVE EAR | >6 | BODY COLOR | ORANGE |
| LENGTH, EAR LEAF | 19 | CROWN COLOR | YELLOW |
| MAX., WIDTH, EAR LEAF | 2.00 | | |
| ANTHOCYANIN, MARGIN | ABSENT | | |
| ANTHOCYANIN, SHEATH | ABSENT | COB | |
| PUBESCENCE, SHEATH | PRESENT | COLOR | RED |
| PUBESCENCE, MARGINS | ABSENT | DIAMETER MIDPOINT | <1 |
| PLANT, TILLERING TASSEL | 0.00 | | |
| | | | |
| TASSEL | | INSECT RESISTANCE | |
| COMPACTNESS | LOOSE | ATTRACTIVE TO APHIDS | NO |
| BRANCH ANGLE | >60 | | |
| NUMBER PRIMARY BRANCHES | >8 | | |
| SECONDARY BRANCH | ABSENT | | |
| LENGTH | >14 | | |
| TASSEL FERTILITY | 1 | | |
| SIZE RATING | 6 | | |
| TASSEL EXTENSION | PARTIALLY ENCLOSED | | |
| SHED IN BOOT? | N/A | | |
| DIFFICULTY IN PULLING | EASY | | |
| NUMBER LEAVES PULLED | 2 | | |
| ANTHER COLOR | YELLOW | | |
| GLUME COLOR - TIP | RED | | |
| - BASE | LIGHT GREEN | | |
| - BAND | DARK GREEN | | |
| POLLEN SHED DURATION | N/A | | |
| POLLEN AMOUNT RATING | AVERAGE | | |

**Table 21**

| **Description of Line AR5253*bm3*** | | | |
|---|---|---|---|
| **AR5253*bm3*** | | | |
| COTYLEDON LEAF | | EAR CHARACTERISTICS | |
| ANTHOCYANIN | PRESENT | SILK COLOR | YELLOW |
| LENGTH | 2-3 | EAR LEAVES | ABSENT |
| | | HUSK | |
| PLANT AND STALK | | COVERAGE | COVERS TIP |
| PLANT HEIGHT | 6.0 | LOOSENESS | N/A |
| EAR HEIGHT | 2.5 | | |
| UNIFORMITY RATING | 5 | EAR ANGLE RATING | 6 |
| ANTHOCY. IN BRACE ROOTS | PRESENT | SHANK LENGTH | 4-12 |
| ANTHOCY. IN NODES | ABSENT | EAR LENGTH | 6-9 |
| DIAM. AT 2nd NODE | 1.1 | EAR SHAPE | CYLINDRICAL |
| ROOT RATING | EXCELLENT | UNIFORMITY | 8 |
| | | | |
| LEAVES | | KERNEL | |
| ANGLE | UPRIGHT | NO. ROWS | 14 |
| COLOR | MEDIUM | TYPE | SEMI DENT |
| TOTAL NUMBER | 10-15 | SIZE | AVERAGE |
| NUMBER ABOVE EAR | 5-6 | BODY COLOR | ORANGE |
| LENGTH, EAR LEAF | 25 | CROWN COLOR | YELLOW |
| MAX., WIDTH, EAR LEAF | 3.00 | | |
| ANTHOCYANIN, MARGIN | ABSENT | | |
| ANTHOCYANIN, SHEATH | PRESENT | COB | |
| PUBESCENCE, SHEATH | PRESENT | COLOR | RED |
| PUBESCENCE, MARGINS | ABSENT | DIAMETER MIDPOINT | 1-2 |
| PLANT, TILLERING TASSEL | 0.00 | | |
| | | | |
| TASSEL | | INSECT RESISTANCE | |
| COMPACTNESS | AVERAGE | ATTRACTIVE TO APHIDS | NO |
| BRANCH ANGLE | 30-60 | | |
| NUMBER PRIMARY BRANCHES | <4 | | |
| SECONDARY BRANCH | PRESENT | | |
| LENGTH | 10-14 | | |
| TASSEL FERTILITY | 3 | | |
| SIZE RATING | 4 | | |
| TASSEL EXTENSION | PARTIALLY ENCLOSED | | |
| SHED IN BOOT? | NO | | |
| DIFFICULTY IN PULLING | EASY | | |
| NUMBER LEAVES PULLED | 1 | | |
| ANTHER COLOR | YELLOW | | |
| GLUME COLOR - TIP | RED | | |
| - BASE | YELLOW | | |
| - BAND | DARK GREEN | | |
| POLLEN SHED DURATION | N\A | | |
| POLLEN AMOUNT RATING | AVERAGE | | |

**Table 22**

| **Description of Line AR5153*bm3*** | | | |
|---|---|---|---|
| **AR5153*bm3*** | | | |
| COTYLEDON LEAF | | EAR CHARACTERISTICS | |
| ANTHOCYANIN | PRESENT | SILK COLOR | YELLOW |
| LENGTH | 2-3 | EAR LEAVES | ABSENT |
| | | HUSK | |
| PLANT AND STALK | | COVERAGE | LONG |
| PLANT HEIGHT | 6.0 | LOOSENESS | N/A |
| EAR HEIGHT | 2.0 | | |
| UNIFORMITY RATING | 6 | EAR ANGLE RATING | 7 |
| ANTHOCY. IN BRACE ROOTS | PRESENT | SHANK LENGTH | <4 |
| ANTHOCY. IN NODES | ABSENT | EAR LENGTH | N/A |
| DIAM. AT 2nd NODE | 1.3 | EAR SHAPE | N/A |
| ROOT RATING | EXCELLENT | UNIFORMITY | 0 |
| | | | |
| LEAVES | | KERNEL | |
| ANGLE | UPRIGHT | NO. ROWS | N/A |
| COLOR | MEDIUM | TYPE | N/A |
| TOTAL NUMBER | 10-15 | SIZE | N/A |
| NUMBER ABOVE EAR | 5-6 | BODY COLOR | N/A |
| LENGTH, EAR LEAF | 28 | CROWN COLOR | N/A |
| MAX., WIDTH, EAR LEAF | 3.50 | | |
| ANTHOCYANIN, MARGIN | ABSENT | | |
| ANTHOCYANIN, SHEATH | ABSENT | COB | |
| PUBESCENCE, SHEATH | ABSENT | COLOR | N/A |
| PUBESCENCE, MARGINS | PRESENT | DIAMETER MIDPOINT | N/A |
| PLANT, TILLERING TASSEL | 0.00 | | |
| | | | |
| TASSEL | | INSECT RESISTANCE | |
| COMPACTNESS | COMPACT | ATTRACTIVE TO APHIDS | NO |
| BRANCH ANGLE | <30 | | |
| NUMBER PRIMARY BRANCHES | 4-6 | | |
| SECONDARY BRANCH | PRESENT | | |
| LENGTH | 10-14 | | |
| TASSEL FERTILITY | 3 | | |
| SIZE RATING | 0 | | |
| TASSEL EXTENSION | PARTIALLY ENCLOSED | | |
| | | | |
| SHED IN BOOT? | YES | | |
| DIFFICULTY IN PULLING | EASY | | |
| NUMBER LEAVES PULLED | 2 | | |
| ANTHER COLOR | YELLOW | | |
| GLUME COLOR - TIP | LIGHT GREEN | | |
| - BASE | LIGHT GREEN | | |
| - BAND | DARK GREEN | | |
| POLLEN SHED DURATION | N/A | | |
| POLLEN AMOUNT RATING | LONG | | |

**Table 23**

| **Description of Line AR5551*bm3*** | | | |
|---|---|---|---|
| **AR5551*bm3*** | | | |
| COTYLEDON LEAF | | EAR CHARACTERISTICS | |
| ANTHOCYANIN | PRESENT | SILK COLOR | YELLOW |
| LENGTH | 2-3 | EAR LEAVES | LONG |
| | | HUSK | |
| PLANT AND STALK | | COVERAGE | SHORT |
| PLANT HEIGHT | 5.5 | LOOSENESS | N/A |
| EAR HEIGHT | 1.5 | | |
| UNIFORMITY RATING | 7 | EAR ANGLE RATING | 5 |
| ANTHOCY. IN BRACE ROOTS | PRESENT | SHANK LENGTH | >12 |
| ANTHOCY. IN NODES | ABSENT | EAR LENGTH | 6-9 |
| DIAM. AT 2nd NODE | 2.9 | EAR SHAPE | CYLINDRICAL |
| ROOT RATING | EXCELLENT | UNIFORMITY | 5 |
| | | | |
| LEAVES | | KERNEL | |
| ANGLE | DROOPING | NO. ROWS | 16 |
| COLOR | MEDIUM | TYPE | SEMI DENT |
| TOTAL NUMBER | 10-15 | SIZE | AVERAGE |
| NUMBER ABOVE EAR | 5-6 | BODY COLOR | ORANGE |
| LENGTH, EAR LEAF | 30 | CROWN COLOR | YELLOW |
| MAX., WIDTH, EAR LEAF | 4.00 | | |
| ANTHOCYANIN, MARGIN | ABSENT | | |
| ANTHOCYANIN, SHEATH | ABSENT | COB | |
| PUBESCENCE, SHEATH | ABSENT | COLOR | WHITE |
| PUBESCENCE, MARGINS | PRESENT | DIAMETER MIDPOINT | 1-2 |
| PLANT, TILLERING TASSEL | 0.00 | | |
| | | | |
| TASSEL | | INSECT RESISTANCE | |
| COMPACTNESS | LOOSE | ATTRACTIVE TO APHIDS | NO |
| BRANCH ANGLE | >60 | | |
| NUMBER PRIMARY BRANCHES | >8 | | |
| SECONDARY BRANCH | ABSENT | | |
| LENGTH | >14 | | |
| TASSEL FERTILITY | 1 | | |
| SIZE RATING | 6 | | |
| TASSEL EXTENSION | PARTIALLY ENCLOSED | | |
| SHED IN BOOT? | NO | | |
| DIFFICULTY IN PULLING | AVERAGE | | |
| NUMBER LEAVES PULLED | 1 | | |
| ANTHER COLOR | YELLOW | | |
| GLUME COLOR - TIP | LIGHT GREEN | | |
| - BASE | LIGHT GREEN | | |
| - BAND | DARK GREEN | | |
| POLLEN SHED DURATION | N/A | | |
| POLLEN AMOUNT RATING | HEAVY | | |

Eleven *bm* hybrids were developed from inbreds homozygous for *bm3*. These hybrids were grown and ensiled at six U.S. locations for 2 years and pairwise comparisons were made between each *bm* hybrid and the corresponding normal isogenic hybrid. The results for forage yield, IVTD and *in vitro* NDF digestibility are summarized in Table 24. Hybrids are listed in Table 24 according to their maturity rating. For example, hybrid number 330659 is a short season (95 day MMR) type, whereas 330670, 330671 and 330672 are long season (115 day MMR) types.

The results indicate that F₁ *bm* hybrids can be developed whose silage has an NDF digestibility of 47% or greater. The results also show that *bm* hybrids can be developed and adapted that have an IVTD of 75% or greater and a forage yield that is only 3-15% less than the yield of the isogenic normal hybrid. For example, hybrid 330662 has a 105 day maturity rating and an average NDF dig. of 51.78%, yet has a forage yield of 25.59 tons/acre. Hybrid 330671 has a 115 day maturity rating and an average NDF dig. of 47.88%, yet has a forage yield of 27.00 tons/acre. Moreover, these results indicate that *bm* hybrids derived from different heterotic groups and adapted to different maturities can be developed. Silage from such *bm* hybrids is suitable for feeding to cattle as disclosed herein.

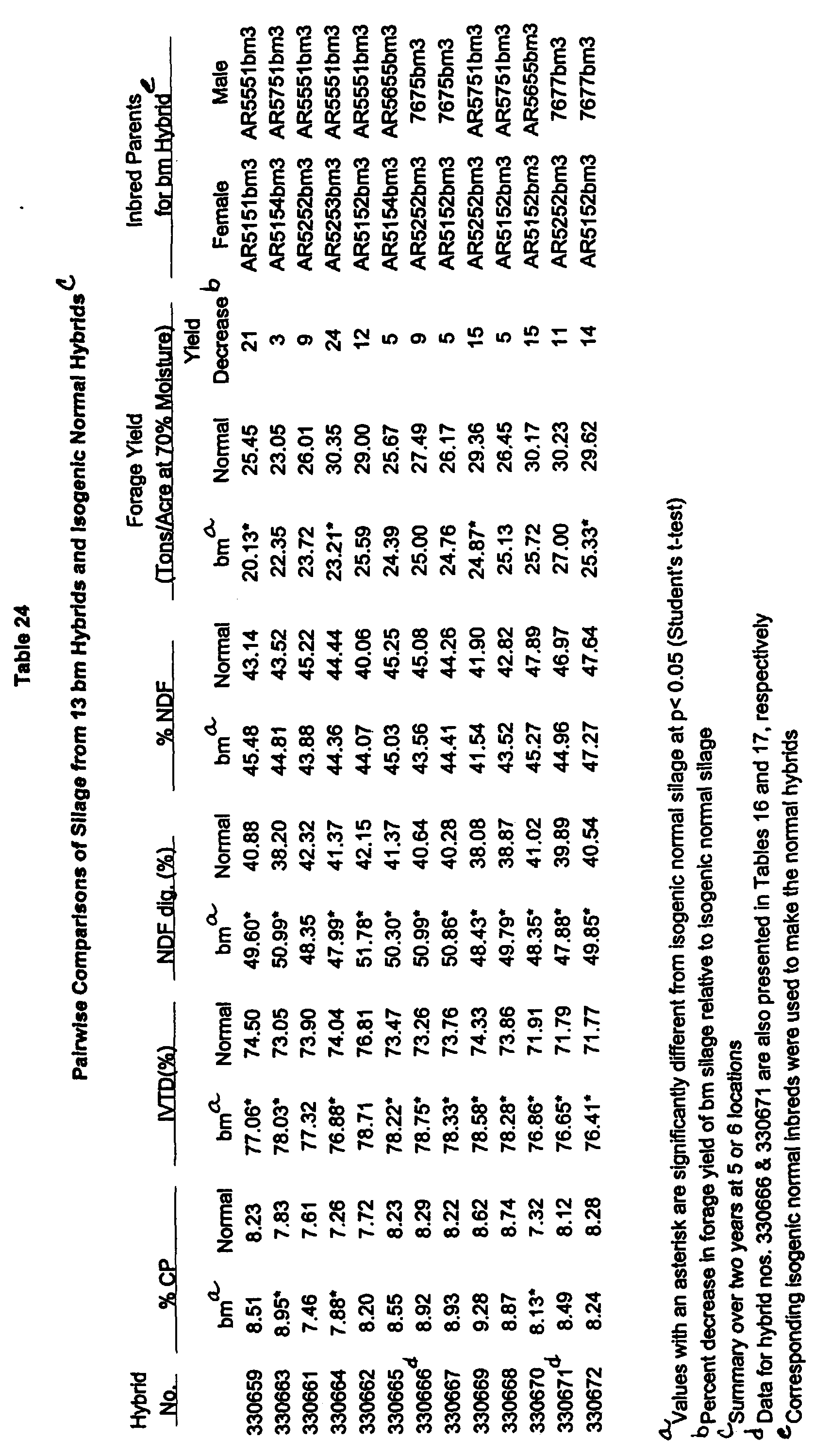

### Example 5

### Formulation of a Dairy Cattle Feed Ration

Control and *bm* F₁ hybrid seed of Example 1 was planted and cultivated in the Midwest United States using a standard agronomic practices. The corn was harvested approximately 5 months after planting, using standard corn silage harvesting methods. The harvested material was ensiled in 150T capacity concrete bunker silos. The harvested material was not treated with inoculant or preservative.

A subsample of each load was ensiled in a 10.16 centimeter (cm) diameter X 40.64 cm length PVC experimental silo fermentation canister made of polyvinyl chloride. Each silo contained a 0.64 cm X 2.54 cm piece of copper tubing inserted into the sidewall. The tubing was covered with a rubber policeman having a slit that allowed fermentation gases to escape while preventing entry of ambient air. Each fresh-cut silage sample was packed tightly into a silo and allowed to ferment for 30 days at ambient temperatures (above freezing) before opening and assaying for nutrient concentrations.

The results of the nutrient concentration assays are shown in Table 25. *In vitro* NDF digestibility was measured as described in Goering, H. and Van Soest, P., *supra,* except that the fermentation period was 30 hours instead of 48 hours. The control corn silage and the BMR corn silage were similar in crude protein and NDF and differed in 30 hour *in vitro NDF* digestibility.

**Table 25**

| **Nutrient composition of corn silages**^{**1**} | | |
|---|---|---|
| Nutrient | Control | BMR |
| Dry matter, % as fed | 33.5 | 30.2 |
| Crude protein, % | 8.35 | 8.68 |
| ADF, % | 21.02 | 21.08 |
| NDF, % | 40.38 | 42.02 |
| Hemicellulose, % | 19.36 | 20.94 |
| NDF dig., % | 36.8 | 45.3 |
| Ash, % | 3.76 | 4.24 |

| | | |
|---|---|---|
| ¹Nutrients and digestibility are expressed on a dry matter basis. | | |

Experimental diets were developed to be fed as a total mixed ration (TMR), using the following assumptions: the early lactation experimental dairy cow was estimated to weigh 613.6 kg and to produce 45.45 kg of milk per day with 3.5% fat, 3.30% milk protein and a 0.18 kg/day increase in body weight. Dry matter intake (DMI) was predicted to be slightly less than 4.0% of body weight.

The two TMRs were formulated to contain 56% forage on a dry matter basis. The forage consisted of 4 parts corn silage (either normal or BMR) to 1 part alfalfa haylage on a dry matter basis. The TMR also contained soybean meal (SBM, 44% crude protein), high moisture corn, finely ground dry corn, whole linted cottonseeds, mineral and vitamins. The two diets were balanced to be isonitrogenous and varied only in the proportions of SBM and high moisture corn. Rations contained approximately 18.0% crude protein, 44% of which was soluble protein and 31% of which was rumen undegraded protein (rumen bypass protein). Both rations were balanced to meet or exceed mineral and vitamin requirements. The ingredient composition of the two rations is given in Table 26. The nutrient composition of the two rations is given in Table 27.

**Table 26**

| **Ingredient Composition of Total Mixed Ration**^{**1**} | | |
|---|---|---|
| INGREDIENT | Normal Silage (% by weight) | BMR Silage (% by weight) |
| Corn silage | 44.6 | 44.6 |
| Alfalfa silage | 11.2 | 11.2 |
| Soybean Meal (44% protein) | 19.2 | 18.8 |
| Corn, high moisture | 8.8 | 9.2 |
| Corn, dry ground | 5.6 | 5.6 |
| Cottonseeds, whole linted | 5.6 | 5.6 |
| Minerals and Vitamins | 5.0 | 5.0 |
| Total | 100.0 | 100.0 |

| | | |
|---|---|---|
| ¹Dry matter basis. | | |

**Table 27**

| **Nutrient Composition of Total Mixed Ration**^{**1**} | | |
|---|---|---|
| NUTRIENT | Normal Silage | BMR Silage |
| Dry Matter | 46.8 | 43.8 |
| Crude Protein (CP) | 18.0 | 18.0 |
| Soluble Protein(SP as % of CP) | 43.8 | 44.2 |
| RUP (RUP as % of CP) | 31.0 | 31.0 |
| Fat | 3.8 | 3.8 |
| Net Energy of Lactation (NE_{L})² Mcal/kg | 1.72 | 1.72 |
| Acid Detergent Fiber (ADF) | 18.4 | 18.5 |
| Neutral Detergent Fiber(NDF) | 30.6 | 31.3 |
| Non-Fiber Carbohydrate (NFC) | 39.6 | 38.8 |
| Calcium | 1.01 | 1.01 |
| Phosphorus | 0.45 | 0.45 |
| Magnesium | 0.29 | 0.29 |
| Sulfur | 0.22 | 0.22 |
| Salt | 0.76 | 0.76 |

| | | |
|---|---|---|
| ¹Percentages for nutrients given as % by weight on a dry matter basis. Dry matter is given as % by weight of ration as fed. | | |
| ²Book values (1.67 Mcal/kg) used for corn silage. | | |

### Example 6

### Milk Production Using BMR Silage in a Dairy Cattle Ration

32 lactating dairy cows were utilized in a complete block design experiment, consisting of 2 primiparous cows and 14 multiparous cows fed a ration containing control silage and 2 primiparous cows and 14 multiparous cows fed BMR silage. Cows within blocks were randomly assigned to treatments. All cows were disease free and otherwise healthy. Rations were formulated as described in Example 5.

The experiment was a crossover design with 28 day periods. The first 21 days of the period were used for diet adjustment and the last 7 days were used for data collection. Cows ranged from approximately 22 to 141 days in milk, averaging 90 days in milk at the beginning of the experiment.

Cows were milked 3 times daily. Milk production was measured at each milking and reported daily. Nine samples of milk were taken on 3 different days from each cow for composition analysis in each collection period. Composition analysis included fat, protein, lactose, and somatic cell counts. Composition analysis was carried out using Near Infrared Spectroscopy at the Michigan Dairy Herd Improvement Association, East Lansing, MI. Body weights were recorded on 2 consecutive days before the experiment started and on the last 2 days of each data collection period. Body condition scores were recorded on the day before the experiment started and on the last day of each data collection period. Scores were recorded according to the definitions indicated in Table 6. Three individuals recorded condition scores at each measurement time.

After 28 days, cows were abruptly switched from one ration to the other ration (from ration containing BMR silage to ration containing control silage and vice versa). Cows were fed and milked in the same manner as prior to the switch in ration. Milk was weighed and analyzed in the same manner as prior to the switch in ration. The experiment was terminated after 56 days. Data were analyzed using the fit model procedure in the JMP computer statistics program (SAS Institute, North Carolina).

The milk production results are shown in Table 28. The results indicate that cows fed BMR silage have a statistically significant increase in milk production when comparing treatment means. The increase was more than 2.7 kg of milk/cow/day on an uncorrected basis and about 2.6 kg milk/cow/day on a solids-corrected basis (Table 28, lines 2 and 5). There was no significant change in the percentage milk fat, protein, lactose, or solids-not-fat (SNF) between milk from cows fed control or BMR silage (Table 28, lines 6-9).

Dry matter intake (DMI) by cows fed BMR silage was significantly greater than that of cows fed control silage (Table 28, line 1). It is noteworthy that there was no significant difference in mean milk production for the two rations when dry matter intake (DMI) was used a covariate (P=0.42). This result suggests that increased milk production is due to an increase in the amount of total ration consumed per day when BMR silage was fed, i.e., that the rate of intake and feed conversion was increased.

Because milk from BMR silage-fed cows has a composition similar to that of milk from control silage-fed cows, milk from BMR-fed cows can be pooled with milk from other sources and processed by standard techniques. Moreover, milk from BMR silage-fed cows typically will not be subject to a price penalty due to non-standard component concentrations.

The effect of a ration comprising BMR silage on body condition and weight is shown in Table 29. Feeding of such a ration resulted in a greater weight gain than did feeding of a ration comprising control silage, although this difference was not statistically significant. Moreover, the body condition score change was significantly better when cows were fed BMR silage than when cows were fed control silage (P=0.049; Table 29). These results indicate that not only does feeding of a ration comprising BMR silage as disclosed herein increase milk production, cows fed such a ration have improved body condition scores. Improved body condition scores are a general measure of a cow's ability to sustain milk production over an extended period of time, e.g., multiple lactations.

**Table 28**

| **Milk Production** | | | |
|---|---|---|---|
| Component | Control Silage | BMR Silage | P Value |
| Dry Matter Intake (DMI)^{e} | 23.5 | 25.5 | <0.0001 |
| Milk Yield^{c} | 39.0 | 41.7 | 0.002 |
| 4% FCM^{a} | 35.7 | 38.0 | 0.0005 |
| 3.5% FCM^{f} | 38.5 | 41.0 | 0.0005 |
| Solids Corrected Milk^{g} | 35.2 | 37.8 | 0.0001 |
| | | | |
| Milk Fat (%)^{d} | 3.46 | 3.43 | 0.71 |
| Milk Protein (%) | 2.95 | 2.99 | 0.35 |
| Milk Lactose (%) | 4.85 | 4.90 | 0.22 |
| SNF^{b} (%) | 8.52 | 8.59 | 0.13 |
| | | | |
| Milk Fat (kg/cow/day) | 1.33 | 1.42 | 0.01 |
| Milk Protein (kg/cow/day) | 1.14 | 1.24 | 0.0001 |
| Milk Lactose (kg/cow/day) | 1.88 | 2.04 | 0.0005 |

| | | | |
|---|---|---|---|
| ^{a} = Milk yield on a fat-corrected basis to 4% milkfat. (Milk x 0.4)+(lbs. fat x 15.0). lbs fat = Milk x fat content. | | | |
| ^{b} = Percentage of Solids-not-fat. | | | |
| ^{c} = Milk production is expressed as kg/cow/day, unless indicated otherwise. | | | |
| ^{d} = Dry matter intake is expressed a kg per cow per day. | | | |
| ^{e} = Milk components are expressed as a percentage (gm/100ml of milk), unless indicated otherwise. | | | |
| ^{f} = Milk yield on a fat-corrected basis to 3.5% milkfat. (Milk x 0.4255)+(16.425 x Fat content/100 x milk). | | | |
| ^{g} = Tyrrell, H.F. and J.J. Reid, J. Dairy Sci. 48:1215 (1965). | | | |

**Table 29**

| **Effect of BMR Silage on Body Condition and Weight Change** | | | |
|---|---|---|---|
| Component | Control Silage | BMR Silage | P Value |
| Body Weight Change (kg increase after 28 days) | 2.2 | 4.76 | 0.45 |
| Body Condition Score Change after 28 days | 0.019 | 0.110 | 0.049 |
| Ratio: 4% FCM/DMI | 1.52 | 1.48 | 0.23 |

### Example 7

### Milk Production Using a BMR Silage Ration and Bovine Somatotropin

An on-farm herd of about 80 lactating dairy cows was utilized to determine the effect of feeding BMR silage in combination with bovine somatotropin (BST) treatment. Cows were fed in a switchback trial, consisting of feeding a control ration for the first phase, feeding a BMR ration for the second phase, followed by a return to a control ration.

BMR corn hybrid 330666 (Table 24), produced from a cross of inbreds AR5252*bm3* X 7675*bm3*, was grown and harvested for silage at the half milk line stage, using typical commercial farm practices. The material was ensiled for about 75 days in an outdoor environment before feeding. The nutrient composition of the silage was similar to that shown in Table 24. Control silage from a locally adapted normal hybrid was grown and harvested in the same manner.

Experimental diets were developed to be fed as a total mixed ration (TMR), assuming a dairy cow 100 days in milk, weighing 613.6 kg and producing 45.45 kg of milk per day with 3.9% fat, 3.30% milk protein. No increase in body weight was predicted. Dry matter intake (DMI) was predicted to be slightly greater than 4.0% of body weight.

The TMRs were formulated to contain about 46-47% forage on a dry matter basis. The forage consisted of approximately equal parts corn silage (normal or BMR) and alfalfa haylage on a dry matter basis. The TMRs also contained heated soybean flakes, high moisture corn, whole linted cottonseeds, and Gro-Mark(tm) Pro-Barley Mix, a proprietary mixture believed to comprise grain, minerals and vitamins. The TMRs were balanced to be isonitrogenous and to meet or exceed mineral and vitamin requirements. Rations contained slightly more than 18% crude protein, about 31-32% of which was soluble protein. The ingredient composition and the nutrient composition of the total mixed rations is given in Tables 30-31.

About 75% of the cows in the herd were treated with BST during the trial. BST was not administered to cows less than 30 days in milk nor to cows not confirmed to be pregnant; such cows constituted about 25% of the herd. BST (Posilac(tm), Monsanto, St. Louis, MO) was administered according to the manufacturer's instructions, using dosages recommended by the manufacturer.

**Table 30**

| **Ingredient Composition of Total Mixed Ration**^{**1**} | | | |
|---|---|---|---|
| INGREDIENT | TMR² | | |
| | Days 1-10 (Control Silage) | Days 11-21 (BMR Silage) | Days 22-31 (Control Silage) |
| Corn silage | 22.57 | 21.52 | 22.16 |
| Alfalfa silage | 24.90 | 25.79 | 23.76 |
| Soybean Flakes, heated | 2.60 | 3.00 | 3.94 |
| Corn, high moisture | 25.05 | 24.92 | 25.36 |
| Cottonseeds, whole linted | 4.60 | 4.59 | 4.66 |
| Gro-Mark(tm) ProBarley Mix | 20.28 | 20.18 | 20.13 |
| Total | 100.0 | 100.0 | 100.0 |

| | | | |
|---|---|---|---|
| ¹Dry matter basis. | | | |
| ²% by weight. | | | |

**Table 31**

| **Nutrient Composition of Total Mixed Ration**^{**1**} | | | |
|---|---|---|---|
| NUTRIENT | TMR | | |
| | Days 1-10 | Days 11-21 | Days 22-31 |
| Dry Matter | 51.62 | 48.58 | 55.41 |
| Crude Protein (CP) | 18.58 | 18.53 | 18.16 |
| Soluble Protein(SP as % of CP) | 32.42 | 32.05 | 30.82 |
| Crude Fat | 5.59 | 5.56 | 5.83 |
| Net Energy of Lactation (NE_{L})² Mcal/kg | 1.72 | 1.72 | 1.74 |
| Acid Detergent Fiber (ADF) | 18.87 | 18.70 | 18.50 |
| Neutral Detergent Fiber(NDF) | 29.43 | 28.92 | 28.81 |
| Calcium | 1.08 | 1.10 | 1.05 |
| Phosphorus | 0.57 | 0.57 | 0.56 |
| Magnesium | 0.34 | 0.35 | 0.34 |
| Sulfur | 0.26 | 0.26 | 0.26 |
| Salt | 0.56 | 0.55 | 0.55 |

| | | | |
|---|---|---|---|
| ¹Percentages for nutrients given as % by weight on a dry matter basis. Dry matter is given as % by weight of ration as fed. | | | |
| ²Book values (1.67 Mcal/kg) used for corn silage. | | | |

Cows ranged from approximately 7 to 489 days in milk, averaging about 211 days in milk at the beginning of the experiment. Cows were milked twice daily. Milk production for the herd was measured at each milking. Cows were fed and milked in the same manner throughout the experiment. On day 1 of the trial, selected cows were treated with BST and the herd was placed on control ration (containing control silage). The herd was abruptly switched from control ration to BMR ration (containing BMR silage) on day 11. On the same day, selected cows were injected with BST. At day 22, cows were abruptly returned to the control ration. At day 25, selected cows were injected with BST.

Herd milk production results are shown in Table 32 and presented graphically in Figure 1. When the herd was fed a control feed ration, average daily milk production of the herd began to increase following treatment of selected cows with BST. Daily milk production peaked about 5-6 days at about 39 kg/cow/day and then declined to about 36 kg/cow/day. A peak in milk production between injections is typical of prolonged release BST administration.

The herd was switched to a ration comprising BMR silage at day 11. In the second phase of the trial, average milk production increased to about 39-40 kg/cow/day at 6 days after the BST treatment and ration change. Milk production remained at about 39-40 kg/cow/day for the duration of this phase of the trial. These results show that delivery of BST to cows in conjunction with the feeding of a ration comprising BMR silage results in a sustained increase in average daily milk production rather than the decline in milk production typically observed when BST treatment alone is used.

The sustained increase in milk production during the BMR ration phase is also shown by comparing the average daily milk production for the last four days of each phase. Average milk production for the last 4 days of the first phase (control ration/BST) was 37.50 kg of milk/cow/day on an uncorrected basis. Average milk production for the last 4 days of the second phase (BMR ration/BST) was 40.45 kg of milk/cow/day on an uncorrected basis.

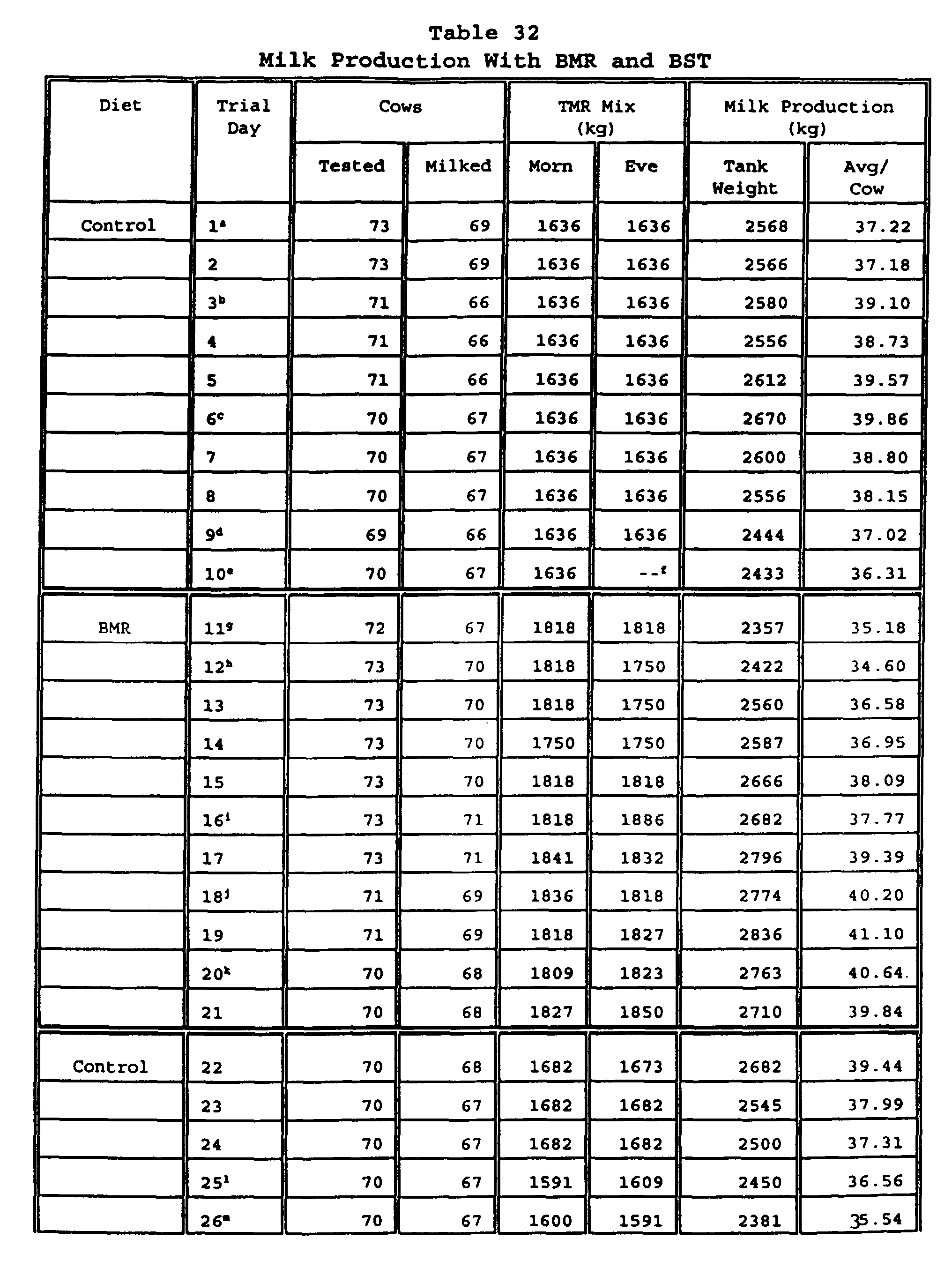

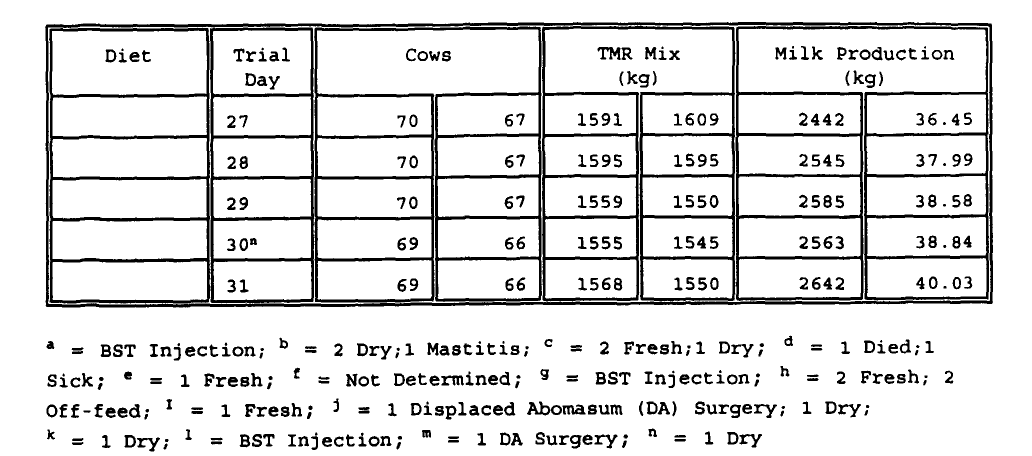

To the extent not already indicated, it will be understood by those of ordinary skill in the art that any one of the various specific embodiments herein described and illustrated may be further modified to incorporate features shown in other of the specific embodiments.

The foregoing detailed description has been provided for a better understanding of the invention only and no unnecessary limitation should be understood therefrom as some modifications will be apparent to those skilled in the art without deviating from the spirit and scope of the appended claims.

## Claims

1. A ruminant animal feed comprising a combination of:
a forage component comprising from about 20% to about 60% of said feed on a dry matter basis, said forage component comprising from about 20% to about 100% corn silage on a dry matter basis produced from corn plants exhibiting a brown midrib (*bm*) phenotype, said silage having an *in vitro* neutral detergent fibre digestibility of about 44% to about 70%; and
a feed composition component;
said animal feed having a fibre content of about 20% to about 40%.

2. A feed as claimed in claim 1, wherein said neutral detergent fibre digestibility is from about 6 percent to about 20 percent greater than the neutral detergent fibre digestibility of corn silage produced from corresponding isogenic normal corn plants.

3. A feed as claimed in claim 1 or 2, wherein said corn plants exhibiting a *(bm)* phenotype comprise F₁ hybrid plants.

4. A feed as claimed in any one of claims 1 to 3, wherein said corn plants are homozygous for *bm3*.

5. A feed as claimed in any one of claims 1 to 4, wherein said silage has a whole plant *in vitro* digestibility from about 65% to about 85%.

6. A feed as claimed in any one of claims 1 to 5, wherein said animal feed has a crude protein content of from about 17% to about 21% on a dry matter basis and about 35% to about 50% of said crude protein is soluble protein.

7. A method of enhancing milk production in a ruminant animal, comprising the step of feeding said animal an animal feed as defined in any one of claims 1 to 6.

8. A method as claimed in claim 7 wherein said animal is a dairy cow.

9. A method as claimed in claim 7 or 8, further comprising the step of administering a biologically active somatotropin to said animal under conditions delivering an effective amount of said somatotropin to said animal during a selected period.

10. A method as claimed in claim 9, wherein said somatotropin is administered as a prolonged release dose.

11. A method as claimed in claim 8 or 9, wherein said conditions comprise delivering said somatotropin to the circulatory system of said animal.

12. A method as claimed in any one of claims 9 to 11, wherein said dose is effective for at least 7 days.

13. A method of producing a total ruminant animal feed as defined in any one of claims 1 to 6 comprising the steps of:
a) obtaining a corn silage produced from corn plants exhibiting a *bm* phenotype; and
b) formulating a total ruminant animal feed comprising a combination of:
a forage component and
a feed composition component as defined in any one of claims 1 to 6.

14. A method as claimed in claim 13 wherein said corn plants are grown from a substantially homogenous assemblage of corn seeds which are homozygous for at least one *bm* allele.

15. A pack for a ruminant animal feed comprising a forage component and separately a feed composition component as defined in any one of claims 1 to 6 as a combined preparation for simultaneous, separate or sequential use to enhance milk production in said animal.

16. A pack as claimed in claim 15 additionally comprising a biologically active somatotropin.
